# EUROPEAN PATENT APPLICATION

(11) **EP 1 947 088 A1**
(43) Date of publication of application: **23.07.2008**
(21) Application number: 07000947.7
(22) Date of filing: 17.01.2007
(51) Int. Cl.: C07D 231/06, A61K 31/415, A61P 3/00

(54) **Substituted pyrazoline compounds with ACAT inhibition activity, their preparation and use as medicaments**

(71) Applicant: Laboratorios del Dr. Esteve S.A., 08041 Barcelona (ES)
(72) Inventor: Yenes-Minguez, Susana, 08750 Molins de Rei (Barcelona) (ES); Torrens-Jover, Antonio, 08221 Tarressa (Barcelona) (ES)
(74) Representative: Graf von Stosch, Andreas

(57) **Abstract**

The present invention relates to substituted pyrazoline compounds, methods for their preparation, medicaments comprising these compounds as well as their use for the preparation of a medicament for the treatment of humans and animals, especially in dyslipidaemia.

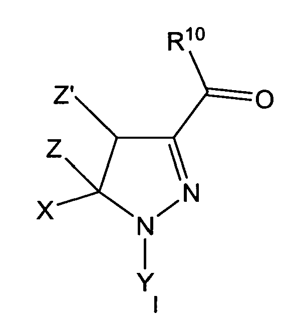

## Description

The present invention relates to substituted pyrazoline compounds, methods for their preparation, medicaments comprising these compounds as well as their use for the preparation of a medicament for the treatment of humans and animals, especially in dyslipidaemia.

It is now abundantly clear that abundant access to highly palatable, calorie-dense, low-cost Westernised diets is producing an explosion in the prevalence of dyslipidaemia, obesity, particularly central adiposity, insulin resistance/impaired glucose tolerance and/or Type 2 diabetes (Zephier et al, 1997; International Diabetes Federation, 2003; Grundy, 2004; Deedwania, 2004). Moreover, when occurring in combination, these cardio-metabolic risk factors comprise the Metabolic Syndrome as defined for example by expert bodies, eg World Health Organisation (WHO, 1999) and the National Cholesterol Education Programme - Third Adult Treatment Panel (NCEP ATP III, 2001) and the International Diabetes Federation (IDF, 2005).

The term "dyslipidaemia" is defined both as a disease entity in its own right and as a component of the Metabolic Syndrome. Thus, within the context of the various definitions of the Metabolic Syndrome, certain lipid abnormalities are specifically defined as follows :

According to world Health Organisation (WHO) (1999) :
- Raised plasma triglycerides (≥ 1.7 mmol/L; 150 mg/dL).
- Low plasma HDL-cholesterol (< 0.9 mmol/L, 35 m/dL men; < 1.0 mmol/L, 39 mg/dL women).

According to National Cholesterol Education Programme (NCEP) Adult Treatment Panel (ATP) III (2002) :
- Raised plasma triglycerides (≥ 1.7 mmol/L; 150 mg/dL).
- Low plasma HDL-cholesterol (< 1.03 mmol/L, 40 mg/dL men; < 1.29 mmol/L, 50 mg/dL women).

According to International Diabetes Federation (IDF) (2005) :
- Raised serum triglycerides (≥ 1.7 mmol/L; 150 mg/dL) or specific treatment for this lipid abnormality.
- Reduced plasma HDL-cholesterol (< 1.03 mmol/L, 40 mg/dL men; 1.29 mmol/L, 50 mg/dL women) or specific treatment for this abnormality.

In addition to the above lipid abnormalities pertaining to definitions of the Metabolic Syndrome, "dyslipidaemia" also encompasses the following disorders as described by the NCEP in their ATP III Guidelines (NCEP ATP III, 2002): heterozygous familial hypercholesterolaemia (FH), homozygous familial hypercholesterolaemia (FH), familial defective apolipoprotein B-100 (FDB), polygenic hypercholesterolaemia hypertriglyceridaemia including familial combined hyperlipidaemia, familial hypertriglyceridaemia and familial dysbetalipoproteinaemia, low HDL-cholesterol (with or without hypertriglyceridaemia), diabetic dyslipidaemia (ie atherogenic dyslipidaemia in persons with Type 2 diabetes), elevated LDL-cholesterol and atherogenic dyslipidaemia. Other secondary dyslipidaemias include, but are not limited to, those evoked by hypothyroidism, nephrotic syndrome and other renal disorders, obstructive liver disease and protease-inhibitor induced dyslipidaemia.

As also stated in the NCEP ATP III Guidelines (2002), the necessity to treat dyslipidaemia in patients is defined not only by the serum or plasma concentrations of individual lipids, but also by the coexistence of lipid abnormalities with other cardiovascular risk factors and/or related disorders, eg hypertension, cardiovascular disease and Type 2 diabetes, the patient's medical history, also whether or not the patient has a history of cerebrovascular or cardiovascular adverse events, eg myocardial infarction, congestive heart failure, angina and/or haemorrhagic or thromboembolic stroke. Such clinical factors are well known to those skilled in the art and are taken into account in the decision whether or not to prescribe medications to treat dyslipidaemia.

In the present invention, all of the above indications are included under the term "dyslipidaemia".

It is also well known that obesity and visceral adiposity are important metabolic consequences of the excessive consumption of highly palatable, calorie-dense foods (Zephier et al, 1997; International Diabetes Federation, 2005; Grundy, 2004; Deedwania, 2004), and in some instances, from cravings for and binge eating, of specific palatable food sources (White et al, 2002; Phelan & Whadden, 2002; Yanovski, 2003; White & Grilo, 2005).

Dyslipaemia, with or without obesity, is major cause of insulin resistance and a key driver of the progression of pre-diabetes (insulin resistance and/or impaired glucose tolerance) to Type 2 diabetes (Boden & Laakso, 2004; Bays et al, 2004; IDF, 2005). Furthermore, there is also a high degree of association between Type 2 diabetes and dyslipidaemia whereby the latter is characterised by raised plasma levels of small, dense LDL-cholesterol particles, elevated triglycerides and low concentrations of HDL-cholesterol particles (Boden & Laakso, 2004).

The Metabolic Syndrome consists of a cluster of cardio-metabolic risk factors, but obesity, central adiposity, lipid abnormalities, (raised serum triglycerides, low serum HDL-cholesterol) and impaired glucose tolerance or Type 2 diabetes are core symptoms of the Metabolic Syndrome, irrespective of whether a diagnosis of is made according to the criteria defined by the World Health Organisation (WHO, 1999), the National Cholesterol Education Programme - Third Adult Treatment Panel (NCEP ATP III, 2001) or the International Diabetes Federation (IDF, 2005).

One Enzyme playing an important role in the metabolism of lipids and, which is thus an interesting target for the effects on dyslipidaemia is Acyl CoA-Cholesterol Acyltransferase (herafter also called in the normal abbreviation: ACAT).

Even though with the pharmaceutical class of statins, which are as such quite potent lipid lowering agents acting by inhibitng HMG-CoA reductase and are useful for the prophylaxis and/or treatment of cardiovascular diseases, there are safety concern related to the use of statins. One of them is the development of rhabdomyolysis, the pathological breakdown of skeletal muscle, which may lead to acute renal failure when muscle breakdown products damage the kidney. Consequently, there is still a big demand for potent therapeutic agents to treat dyslipidaemia, possibly, showing less incidents of undesired side effects of statins, or at least less pronounced.

Thus, it was an object of the present invention to provide novel compounds for use as active substances in medicaments. In particular, these active substances should act on or inhibit ACAT and thus be suitable for the treatment of dyslipidaemia.

Said object was achieved by providing the substituted pyrazoline compounds of general formula I given below, their stereoisomers, corresponding salts and corresponding solvates thereof.

It has been found that these compounds show a high inhibition of ACAT and therefore seem suitable for the prophylaxis and/or treatment of dyslipidaemia, but also diabetes Type II, Metabolic Syndrome or obesity.

Thus, in one of its aspects the present invention relates to a substituted pyrazoline compound of general formula I, 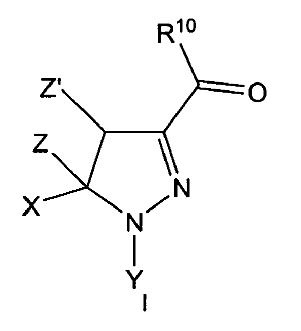 wherein
Z is C₁₋₄-Alkyl, substituted or unsubstituted, branched or linear, saturated or unsaturated;
Z' is selected from hydrogen; C₁₋₄-Alkyl, substituted or unsubstituted, branched or linear, saturated or unsaturated;
X and Y independently represent an optionally at least monsubstituted mono- or polycyclic ring-system;
R¹⁰ represents OR^{8'} or NR⁸R⁹, with
R^{8'} representing a hydrogen atom or a branched or linear C₁₋₄-alkyl group, or
either
R⁸ representing a hydrogen atom or a branched or linear C₁₋₃-alkyl group, while R⁹ is representing an optionally at least monsubstituted mono- or polycyclic ring-system;
   or
R⁸ and R⁹ together with the connecting Nitrogen atom are representing an optionally at least monsubstituted heterocyclyl radical;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

Formula I is also covering the diastereoisomers and thus may also be selected from any of the 4 formulas la, Ib, Ic or Id (clockwise starting in the top left corner) set out below:

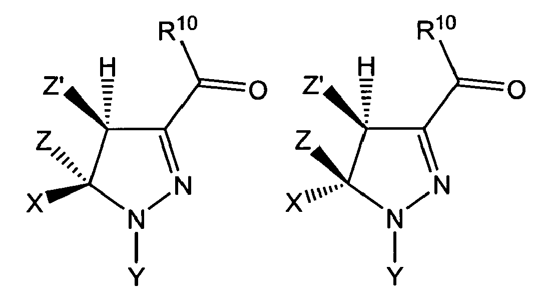

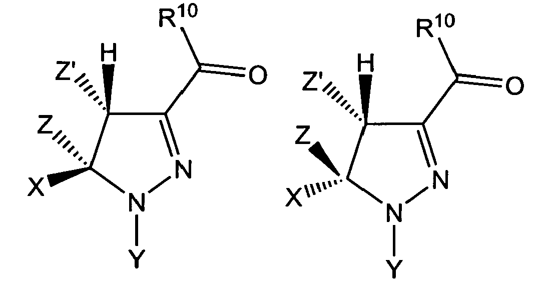

A "mono- or polycyclic ring-system" according to the present invention means a mono- or polycyclic hydrocarbon ring-system that may be saturated, unsaturated or aromatic. If the ring system is polycyclic, each of its different rings may show a different degree of saturation, i.e. it may be saturated, unsaturated or aromatic. Optionally each of the rings of the mono- or polycyclic ring system may contain one or more heteroatoms as ring members, which may be identical or different and which can preferably be selected from the group consisting of N, O, S and P, more preferably be selected from the group consisting of N, O and S. Preferably the polycyclic ring-system may comprise two rings that are condensed. The rings of the mono- or polycyclic ring-sytem are preferably 5- or 6-membered.

An "aryl", "aryl radical" or group is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

In the context of this invention "cycloalkyl radical" or group is understood as meaning saturated and unsaturated (but not aromatic) cyclic hydrocarbons (without a heteroatom in the ring), which can be unsubstituted or mono- or polysubstituted. Furthermore, C₃₋₄-cycloalkyl represents C₃- or C₄-cycloalkyl, C₃₋₅-cycloalkyl represents C₃-, C₄- or C₅-cycloalkyl, C₃₋₆-cycloalkyl represents C₃-, C₄-, C₅- or C₆-cycloalkyl, C₃₋₇-cycloalkyl represents C₃-, C₄-, C₅-, C₆- or C₇-cycloalkyl, C₃₋₈-cycloalkyl represents C₃-, C₄-, C₅-, C₆-, C₇- or C₈-cycloalkyl, C₄₋₅-cycloalkyl represents C₄- or C₅-cycloalkyl, C₄₋₆-cycloalkyl represents C₄-, C₅- or C₆-cycloalkyl, C₄₋₇-cycloalkyl represents C₄-, C₅-, C₆- or C₇-cycloalkyl, C₄₋₈-cycloalkyl represents C₄-, C₅-, C₆- C₇- or C₈-cycloalkyl C₅₋₆-cycloalkyl represents C₅- or C₆-cycloalkyl and C₅₋₇-cycloalkyl represents C₅-, C₆- or C₇-cycloalkyl. However, mono- or polyunsaturated, preferably monounsaturated, cycloalkyls also in particular fall under the term cycloalkyl as long as the cycloalkyl is not an aromatic system. The cycloalkyl radicals are preferably cyclopropyl, 2-methylcyclopropyl, cyclopropylmethyl, cyclobutyl, cyclopentyl, cyclopentylmethyl, cyclohexyl, cycloheptyl, cyclooctyl, and also adamantyl.

A "heterocyclyl", a "heterocyclyl radical" or group or "heterocyclic ring system" is understood as meaning heterocyclic ring systems which contain one or more heteroatoms from the group consisting of nitrogen, oxygen and/or sulfur in the ring or ringsystem, and can also be mono- or polysubstituted. The ringsystem may consist either of only one saturated or unsaturated or even aromatic ring or may consist of 2, 3 or 4 saturated or unsaturated or even aromatic rings, which are condensed in that between two or more of the rings ring members are shared. Examples which may be mentioned from the group of heterocyclyls are furan, benzofuran, thiophene, benzothiophene, pyrrole, pyridine, pyrimidine, pyrazine, quinoline, isoquinoline, phthalazine, benzo-1,2,5-thiadiazole, imidazo-thiazole, benzothiazole, indole, benzotriazole, benzodioxolane, benzodioxane, carbazole and quinazoline.

In connection with mono- or polycyclic ring-system, aryl radical, cycloalkyl radical, or heterocyclyl radical, "substituted" is understood - unless defined otherwise - as meaning replacement of at least one hydrogen radical on the ring-system of the mono- or polycyclic ring-system, the aryl radical, the cycloalkyl radical, or the heterocyclyl radical by OH, SH, =O, halogen (F, Cl, Br, I), CN, NO₂, COOH; NRₓR_{y}, with Rₓ and Ry independently being either H or a saturated or unsaturated, linear or branched, substituted or unsubstituted C₁₋₆-alkyl; by a saturated or unsaturated, linear or branched, substituted or unsubstituted C₁₋₆-alkyl; a saturated or unsaturated, linear or branched, substituted or unsubstituted -O-C₁₋₆₋alkyl (alkoxy); a saturated or unsaturated, linear or branched, substituted or unsubstituted -S-C₁₋₆-alkyl; a saturated or unsaturated, linear or branched, substituted or unsubstituted -C(O)-C₁₋₆-alkyl; a saturated or unsaturated, linear or branched, substituted or unsubstituted -C(O)-O-C₁-6-alkyl; a substituted or unsubstituted phenyl. Within that "monosubstituted" means the substitution of exactly one hydrogen radical, whereas "polysubstituted" means the substitution of more than one hydrogen radical with "polysubstituted"radicals being understood as meaning that the replacement takes effect both on different and on the same atoms several times with the same or different substituents. Therefore, "optionally at least monsubstituted" means either "not substituted" (which is the same as "unsubstituted") if the option is not fulfilled, "monosubstituted" or "polysubstituted".

In connection with aryl radical, cycloalkyl radical, or heterocyclyl radical, "condensed with" is understood as meaning that the ring-system of the aryl radical, the cycloalkyl radical, or the heterocyclyl radical is sharing two atoms (one) of its ring(s) with a ring of the mono- or polycyclic ring-system it is condensed with.

In the context of this invention, "alkyl", "alkyl radical" or group is understood as meaning saturated, linear or branched hydrocarbons, which can be unsubstituted or mono- or polysubstituted. Thus unsaturated alkyl is understood to encompass alkenyl and alkinyl groups, like e.g. -CH=CH-CH₃ or -C≡C-CH₃, while saturated alkyl encompasses e.g. -CH₃ and -CH₂-CH₃. In these radicals, C₁₋₂-alkyl represents C₁- or C₂-alkyl, C₁₋₃-alkyl represents C₁-, C₂- or C₃-alkyl, C₁₋₄-alkyl represents C₁-, C₂-, C₃- or C₄-alkyl, C₁₋₅-alkyl represents C₁-, C₂-, C₃-, C₄-, or C₅-alkyl, C₁₋₆-alkyl represents C₁-, C₂-, C₃-, C₄-, C₅- or C₆-alkyl, C₁₋₇-alkyl represents C₁-, C₂-, C₃-, C₄-, C₅-, C₆- or C₇-alkyl, C₁₋₈-alkyl represents C₁-, C₂-, C₃-, C₄-, C₅-, C₆-, C₇- or C₈-alkyl, C₁₋₁₀-alkyl represents C₁-, C₂-, C₃-, C₄-, C₅-, C₆-, C₇-, C₈-, C₉- or C₁₀-alkyl and C₁₋₁₈-alkyl represents C₁-, C₂-, C₃-, C₄-, C₅-, C₆-, C₇-, C₈-, C₉-, C₁₀-, C₁₁-, C₁₂-, C₁₃-, C₁₄-, C₁₅-, C₁₆-, C₁₇- or C₁₈-alkyl. The alkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also CHF₂, CF₃ or CH₂OH etc.

In connection with alkylene, or alkyl radical or group - unless defined otherwise - the term "substituted" in the context of this invention is understood as meaning replacement of at least one hydrogen radical by F, Cl, Br, I, NH₂, SH or OH; within that "monosubstituted" means the substitution of exactly one hydrogen radical, whereas "polysubstituted" means the substitution of more than one hydrogen radical with "polysubstituted"radicals being understood as meaning that the replacement takes effect both on different and on the same atoms several times with the same or different substituents, for example three times on the same C atom, as in the case of CF₃, or at different places, as in the case of e.g. -CH(OH)-CH=CH-CHCl₂. Therefore, "optionally at least monsubstituted" means either "not substituted" if the option is not fulfilled, "monosubstituted" or "polysubstituted".

The term "alkylene" is understood as meaning a divalent alkyl group like -CH₂- or -CH₂-CH₂-, with (CH₂)₃₋₆ being understood as meaning -CH₂-CH₂-CH₂-, -CH₂-CH₂-CH₂-CH₂-, -CH₂-CH₂-CH₂-CH₂-CH₂- and -CH₂-CH₂-CH₂-CH₂-CH₂-CH₂-, (CH₂)₁₋₄ is to be understood as meaning-CH₂-, -CH₂-CH₂-, -CH₂-CH₂-CH₂- and -CH₂-CH₂-CH₂-CH₂-, (CH₂)₄₋₅ is to be understood as meaning -CH₂-CH₂-CH₂-CH₂- and -CH₂-CH₂-CH₂-CH₂-CH₂-, etc. An "alkylene" may also be unsaturated.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" means in the context of this invention any salt that is physiologically tolerated (most of the time meaning not being toxic- especially not caused by the counter-ion) if used appropriately for a treatment especially if used on or applied to humans and/or mammals.
These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The compounds of the invention may be in crystalline form or either as free compounds or as solvates and it is intended that those forms are within the scope of the present invention. Methods of solvation are generally known within the art. Suitable solvates are pharmaceutically acceptable solvates. The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I) or, or of its salts, solvates or prodrugs.

In a preferred embodiment of the invention the substituted pyrazoline compounds of the invention are compounds according to general formula I, wherein
Z is C₁₋₄-Alkyl, substituted or unsubstituted, branched or linear, saturated or unsaturated;
Z' is selected from hydrogen; C₁₋₄-Alkyl, substituted or unsubstituted, branched or linear, saturated or unsaturated;
X and Y independently represent an aryl radical, cycloalkyl radical, or heterocyclyl radical, which groups are unsubstituted or may be substituted with 1, 2 or 3 substituents W, which can be the same or different, selected from the group branched or linear C₁₋₃-alkyl or branched or linear C₁₋₃-alkoxy, phenyl, hydroxy, chloro, bromo, fluoro, iodo, SH, trifluoromethyl, CHF₂, CH₂F, OCHF₂, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
R¹⁰ represents OR^{8'} or NR⁸R⁹, with
   R^{8'} representing a hydrogen atom or a branched or linear C₁₋₄-alkyl group, or
      either
   R⁸ representing a hydrogen atom or a branched or linear C₁₋₃-alkyl group, while R⁹ is representing an aryl radical, cycloalkyl radical, or heterocyclyl radical, which groups are unsubstituted or may be substituted with; R⁵, R⁶ and
   R⁷, which can be the same or different,
      with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂;
      or
   R⁸ and R⁹ together with the connecting Nitrogen atom are representing an optionally at least monsubstituted heterocyclyl radical; which group is unsubstituted or may be substituted with R⁵, R⁶ and R⁷, which can be the same or different,
      with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

In a preferred embodiment of the invention the substituted pyrazoline compounds of the invention are compounds according to general formula I, wherein
Z is C₁₋₄-Alkyl, substituted or unsubstituted, branched or linear, saturated or unsaturated;
Z' is selected from hydrogen; C₁₋₄-Alkyl, substituted or unsubstituted, branched or linear, saturated or unsaturated;
X and Y independently represent an phenyl, thienyl, naphtyl or pyridyl, which groups are unsubstituted or may be substituted with 1, 2 or 3 substituents W, which can be the same or different, selected from the group
   branched or linear C₁₋₃-alkyl or branched or linear C₁₋₃-alkoxy, phenyl, hydroxy, chloro, bromo, fluoro, iodo, SH, trifluoromethyl, CHF₂, CH₂F, OCHF₂, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
R¹⁰ represents OR^{8'} or NR⁸R⁹, with
   R^{8'} representing a hydrogen atom or a branched or linear C₁₋₄-alkyl group, or
   either
   R⁸ representing a hydrogen atom or a branched or linear C₁₋₃-alkyl group, while R⁹ is representing an aryl radical, cycloalkyl radical, or heterocyclyl radical, which groups are unsubstituted or may be substituted with; R⁵, R⁶ and R⁷, which can be the same or different,
      with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂;
   or
   R⁸ and R⁹ together with the connecting Nitrogen atom are representing an optionally at least monsubstituted heterocyclyl radical; which group is unsubstituted or may be substituted with R⁵, R⁶ and R⁷, which can be the same or different,
      with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

In a preferred embodiment of the invention the substituted pyrazoline compounds of the invention are compounds according to general formula I, wherein
Z is CH₃ or C₂H₅; and/or
Z' is hydrogen; and/or
X and Y independently represent phenyl or thienyl; preferably Y representing phenyl, while X represents phenyl or thienyl; more preferably X and Y representing phenyl; and/or
R¹⁰ represents OR^{8'} with
   R^{8'} representing a hydrogen atom or a branched or linear C₁₋₄-alkyl group, preferably hydrogen, CH₃ or C₂H₅;
      or
R¹⁰ represents NR⁸R⁹, with
   R⁸ representing a hydrogen atom or a branched or linear C₁₋₃-alkyl group, preferably hydrogen; and
   R⁹ is representing an aryl radical, cycloalkyl radical, or heterocyclyl radical;
      or
   R⁸ and R⁹ together with the connecting Nitrogen atom are representing an optionally at least monsubstituted heterocyclyl radical.

In a preferred embodiment of the invention the substituted pyrazoline compounds of the invention are compounds according to general formula II 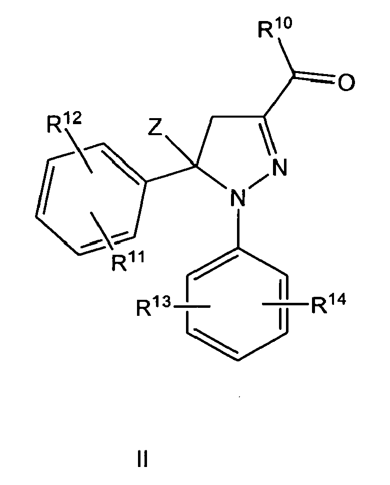 wherein
Z is C₁₋₄-Alkyl, substituted or unsubstituted, branched or linear, saturated or unsaturated;
R¹⁰ represents OR^{8'} or NR⁸R⁹, with
R^{8'} representing a hydrogen atom or a branched or linear C₁₋₄-alkyl group, or
   either
R⁸ representing a hydrogen atom or a branched or linear C₁₋₃-alkyl group, while R⁹ is representing an aryl radical, cycloalkyl radical, or heterocyclyl radical, which groups are unsubstituted or may be substituted with; R⁵, R⁶ and R⁷, which can be the same or different,
   with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂;
   or
R⁸ and R⁹ together with the connecting Nitrogen atom are representing an optionally at least monsubstituted heterocyclyl radical; which group is unsubstituted or may be substituted with R⁵, R⁶ and R⁷, which can be the same or different,
   with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂;
R¹¹, R¹², R¹³ and R¹⁴ independently of one another represent:
H; branched or linear C₁₋₃-alkyl or branched or linear C₁₋₃-alkoxy, phenyl, hydroxy, chloro, bromo, fluoro, iodo, SH, trifluoromethyl, CHF₂, CH₂F, OCHF₂, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

As the general formula II also covers the enantiomers it may also be in the form of general formulas IIa and IIb.

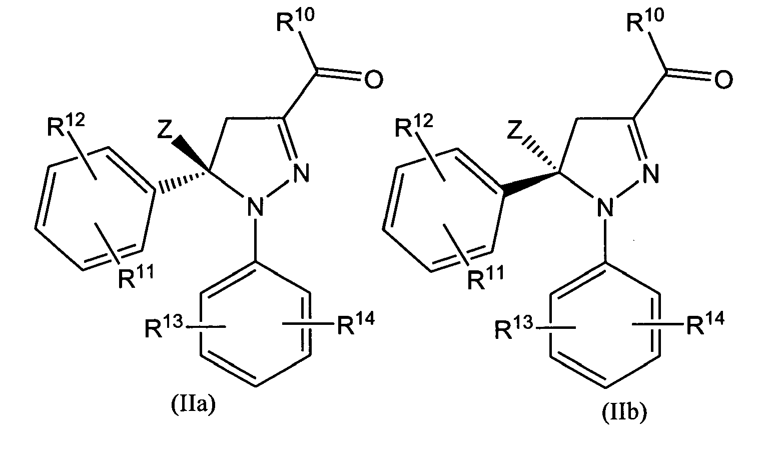

In another preferred embodiment of the invention the substituted pyrazoline compounds of the invention are compounds according to general formula II, wherein
R¹¹, R¹², R¹³ and R¹⁴ independently of one another represent H, CH₃, C₂H₅, C₃H₇, OCH₃, OC₂H₅, OH, SH, F, Cl, Br, I CF₃, CHF₂, CH₂F, OCF₃, OCHF₂; preferably R¹¹, R¹², R¹³ and R¹⁴ independently of one another represent H, OH, OCH₃, F, Cl, Br, I, CF₃, CHF₂ or OCF₃; and/or
Z is CH₃ or C₂H₅; and/or
R¹⁰ represents OR^{8'} with
   R^{8'} representing a hydrogen atom or a branched or linear C₁₋₄-alkyl group, preferably hydrogen, CH₃ or C₂H₅;
   or
R¹⁰ represents NR⁸R⁹, with
   R⁸ representing a hydrogen atom or a branched or linear C₁₋₃-alkyl group, preferably hydrogen; and
   R⁹ is representing an aryl radical, cycloalkyl radical, or heterocyclyl radical;
      or
   R⁸ and R⁹ together with the connecting Nitrogen atom are representing an optionally at least monsubstituted heterocyclyl radical.

In a preferred embodiment of the invention the substituted pyrazoline compounds of the invention are compounds according to general formula III 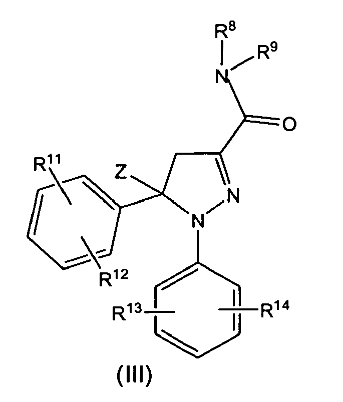 wherein
Z is C₁₋₄-Alkyl, substituted or unsubstituted, branched or linear, saturated or unsaturated;
R⁸ represents a hydrogen atom or a branched or linear C₁₋₃-alkyl group, while R⁹ is representing an aryl radical, cycloalkyl radical, or heterocyclyl radical, which groups are unsubstituted or may be substituted with; R⁵, R⁶ and R⁷, which can be the same or different,
with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂;
or
R⁸ and R⁹ together with the connecting Nitrogen atom are representing an optionally at least monsubstituted heterocyclyl radical; which group is unsubstituted or may be substituted with R⁵, R⁶ and R⁷, which can be the same or different,
with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂;
R¹¹, R¹², R¹³ and R¹⁴ independently of one another represent:
H; branched or linear C₁₋₃-alkyl or branched or linear C₁₋₃-alkoxy, phenyl, hydroxy, chloro, bromo, fluoro, iodo, SH, trifluoromethyl, CHF₂, CH₂F, OCHF₂,trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

As the general formula III also covers the enantiomers it may also be in the form of general formulas IIIa and IIIb:

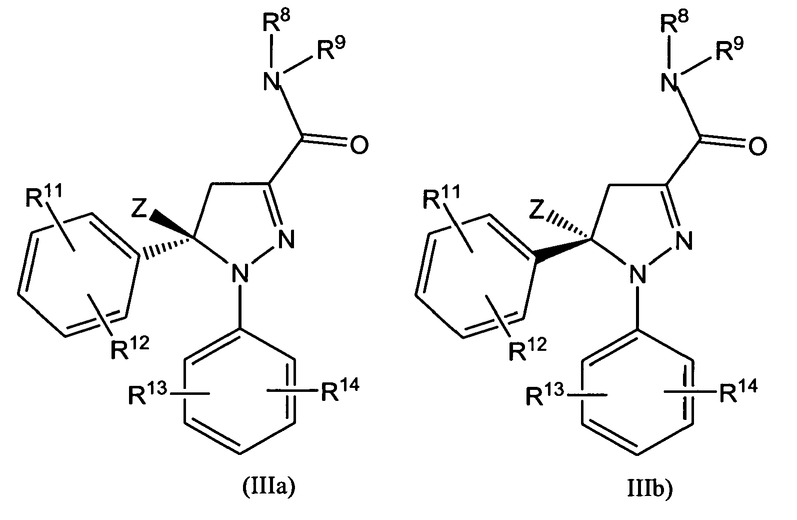

In a preferred embodiment of the invention the substituted pyrazoline compounds of the invention are compounds according to general formula III, wherein
R¹¹, R¹², R¹³ and R¹⁴ independently of one another represent H, CH₃, C₂H₅, C₃H₇, OCH₃, OC₂H₅, OH, SH, F, Cl, Br, I CF₃, CHF₂, CH₂F, OCF₃, OCHF₂; preferably R¹¹, R¹², R¹³ and R¹⁴ independently of one another represent H, OH, OCH₃, F, Cl, Br, I, CF₃, CHF₂ or OCF₃; and/or
Z is CH₃ or C₂H₅; and/or
R⁸ represents a hydrogen atom;
R⁹ represents an aryl radical, cycloalkyl radical, or heterocyclyl radical, which groups are unsubstituted or may be substituted with; R⁵, R⁶ and R⁷, which can be the same or different,
   with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂.

In another preferred embodiment of the invention the substituted pyrazoline compounds of the invention are compounds according to general formula IV 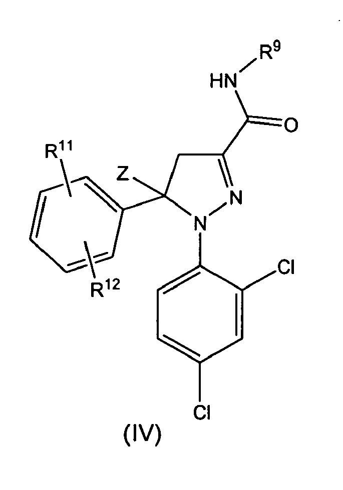 wherein
Z is C₁₋₄-Alkyl, substituted or unsubstituted, branched or linear, saturated or unsaturated;
R⁹ represents an aryl radical, cycloalkyl radical, or heterocyclyl radical, which groups are unsubstituted or may be substituted with; R⁵, R⁶ and R⁷, which can be the same or different,
with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂;
R¹¹ and R¹² independently of one another represent:
H; branched or linear C₁₋₃-alkyl or branched or linear C₁₋₃-alkoxy, phenyl, hydroxy, chloro, bromo, fluoro, iodo, SH, trifluoromethyl, CHF₂, CH₂F, OCHF₂, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

As the general formula II also covers the enantiomers it may also be in the form of general formulas IVa and IVb.

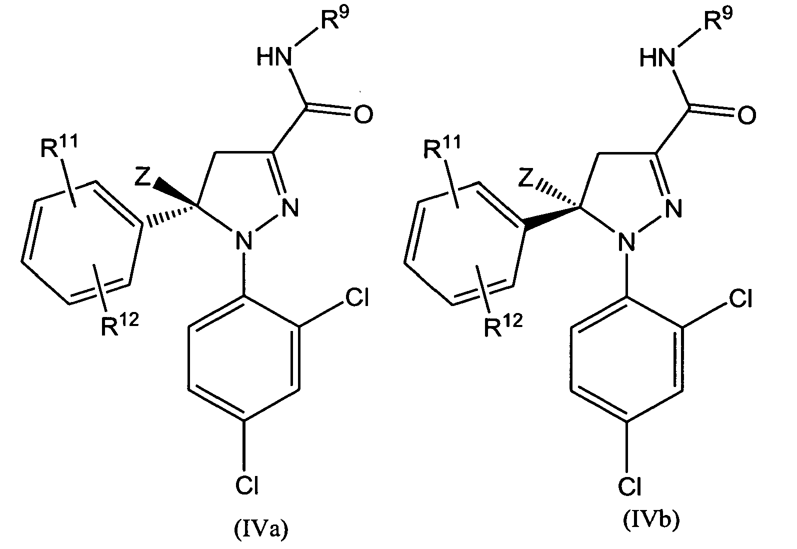

In another preferred embodiment of the invention the substituted pyrazoline compounds of the invention are compounds according to general formula IV, wherein
R¹¹ and R¹² independently of one another represent H, CH₃, C₂H₅, C₃H₇, OCH₃, OC₂H₅, OH, SH, F, Cl, Br, I CF₃, CHF₂, CH₂F, OCF₃, OCHF₂; preferably R¹¹, R¹², R¹³ and R¹⁴ independently of one another represent H, OH, OCH₃, F, Cl, Br, I, CF₃, CHF₂ or OCF₃; and/or
Z is CH₃ or C₂H₅; and/or
R⁹ represents an aryl radical, cycloalkyl radical, or heterocyclyl radical, which groups are unsubstituted or may be substituted with; R⁵, R⁶ and R⁷, which can be the same or different,
   with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂.

In another preferred embodiment of the invention the substituted pyrazoline compounds of the invention are compounds according to general formula IV, wherein
R¹¹ and R¹² independently of one another represent H, CH₃, C₂H₅, C₃H₇, OCH₃, OC₂H₅, OH, SH, F, Cl, Br, I CF₃, CHF₂, CH₂F, OCF₃, OCHF₂; preferably represent H, OH, OCH₃, F, Cl, Br, I, CF₃, CHF₂ or OCF₃; more preferably represent Br, Cl, OH, OCH₃, or H; most preferably represent H; and/or
Z is CH₃ or C₂H₅; preferably is CH₃; and/or
R⁹ represents an aryl radical, cycloalkyl radical, or heterocyclyl radical, which groups are unsubstituted or may be substituted with; R⁵, R⁶ and R⁷, which can be the same or different,
   with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂;
preferably represents an unsubstituted aryl radical, cycloalkyl radical, or heterocyclyl radical.

In another preferred embodiment of the invention the substituted pyrazoline compounds of the invention are compounds according to general formula IV, wherein
R¹¹ and R¹² independently of one another represent H, CH₃, C₂H₅, C₃H₇, OCH₃, OC₂H₅, OH, SH, F, Cl, Br, I CF₃, CHF₂, CH₂F, OCF₃, OCHF₂; preferably represent H, OH, OCH₃, F, Cl, Br, I, CF₃, CHF₂ or OCF₃; more preferably represent Br, Cl, OH, OCH₃, or H; most preferably represent H; and
Z is CH₃ or C₂H₅; preferably is CH₃; and
R⁹ represents
   a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl and bicyclo[2.2.1]heptyl, which may be bonded via a -(CH₂)-, -(CH₂)-(CH₂)-, -(CH₂)-(CH₂)-(CH₂)- or -CH=CH-group and/or may optionally be substituted with 1, 2, 3, 4 or 5 substituent(s) independently selected from the group consisting of - OH, methyl, ethyl, n-propyl, isopropyl, n-butyl, isobutyl, 2-butyl, tert-butyl, n-pentyl, 2-pentyl and n-hexyl;
   a radical selected from the group consisting of phenyl, naphthyl, pyridinyl, furyl (furanyl), thienyl (thiophenyl) and triazolyl, which may be bonded via a -(CH₂)-, -(CH₂)-(CH₂)-, -(CH₂)-(CH₂)-(CH₂)- or -CH=CH-group and/or may optionally be substituted with 1, 2, 3, 4 or 5 substituent(s) independently selected from the group consisting of -CF₃, methyl, ethyl, n-propyl, isopropyl, n-butyl, isobutyl, 2-butyl, tert-butyl, n-pentyl, 2-pentyl, n-hexyl, F, Cl and Br;
or a radical selected from the group consisting of 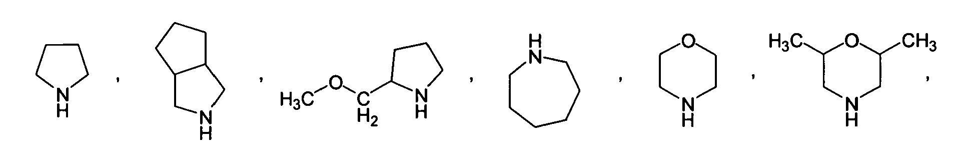 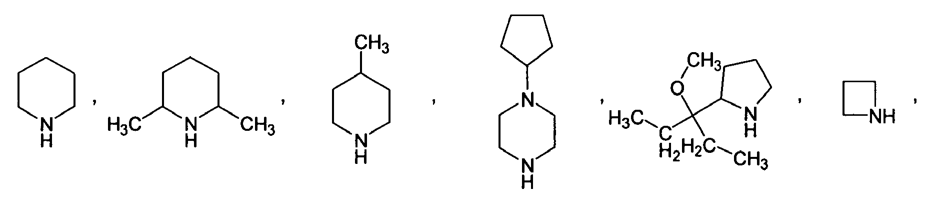 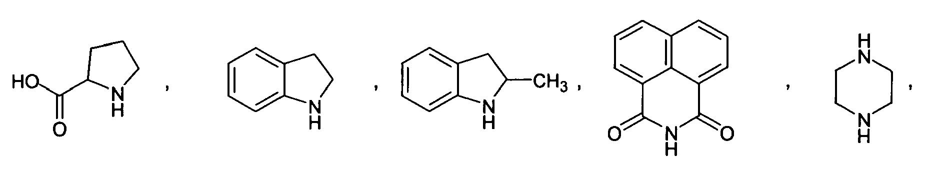 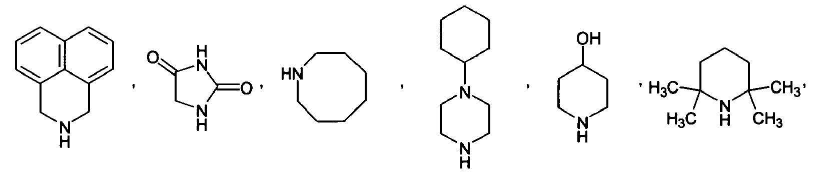 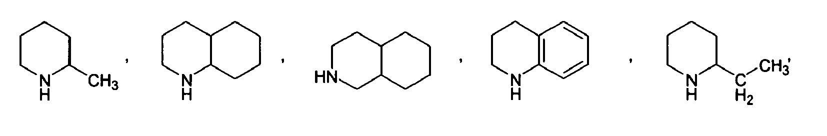 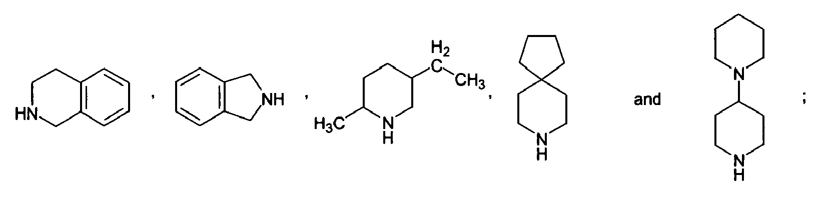 which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals; preferably R⁹ represents a radical selected from the group consisting of phenyl, cyclohexyl, cyclopentyl, cycloheptyl, cyclooctyl or of the group consisting of 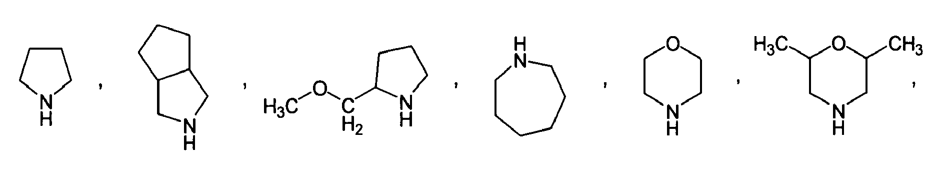 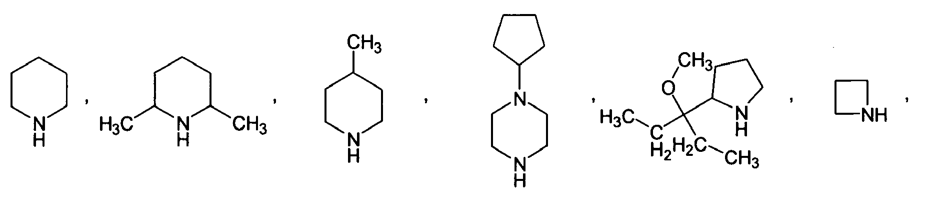 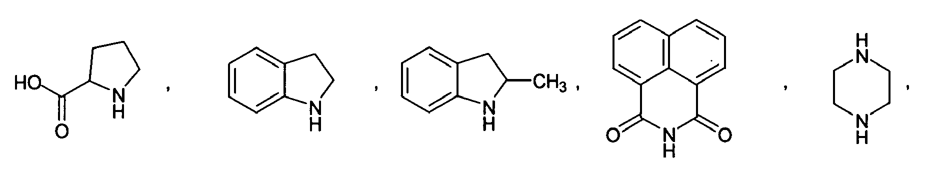 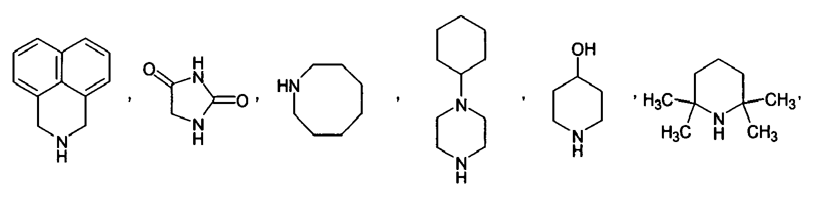 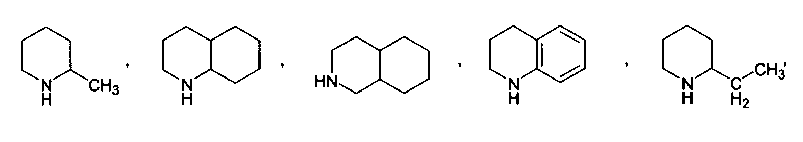 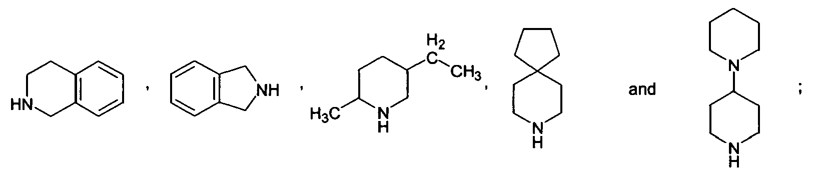 which is in each case bonded to the pyrazoline compound of general formula I at the nitrogen atom of the cyclic part of the aforementioned radicals;
more preferably R⁹ represents
a radical selected from the group consisting of phenyl, cyclohexyl, cyclopentyl, cycloheptyl, cyclooctyl or from the group of 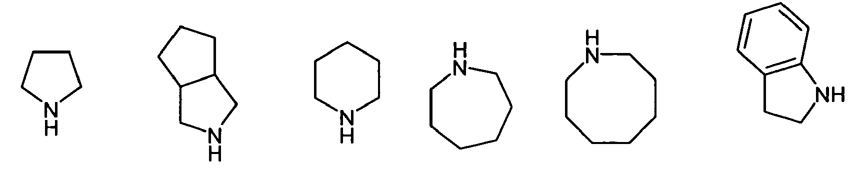 which is in each case bonded to the pyrazoline compound of general formula I at the nitrogen atom of the cyclic part of the aforementioned radicals.

In another preferred embodiment of the invention the substituted pyrazoline compounds of the invention are compounds according to general formula I, II, III, and IV, wherein
R⁹ represents
a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl and bicyclo[2.2.1]heptyl, which may be bonded via a -(CH₂)-, -(CH₂)-(CH₂)-, -(CH₂)-(CH₂)-(CH₂)- or -CH=CH-group and/or may optionally be substituted with 1, 2, 3, 4 or 5 substituent(s) independently selected from the group consisting of - OH, methyl, ethyl, n-propyl, isopropyl, n-butyl, isobutyl, 2-butyl, tert-butyl, n-pentyl, 2-pentyl and n-hexyl;
a radical selected from the group consisting of phenyl, naphthyl, pyridinyl, furyl (furanyl), thienyl (thiophenyl) and triazolyl, which may be bonded via a -(CH₂)-, -(CH₂)-(CH₂)-, -(CH₂)-(CH₂)-(CH₂)- or -CH=CH-group and/or may optionally be substituted with 1, 2, 3, 4 or 5 substituent(s) independently selected from the group consisting of -CF₃, methyl, ethyl, n-propyl, isopropyl, n-butyl, isobutyl, 2-butyl, tert-butyl, n-pentyl, 2-pentyl, n-hexyl, F, Cl and Br;
or a radical selected from the group consisting of 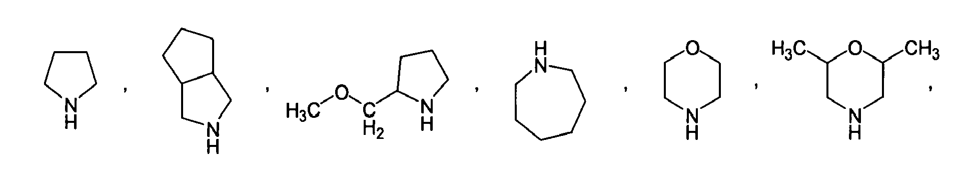 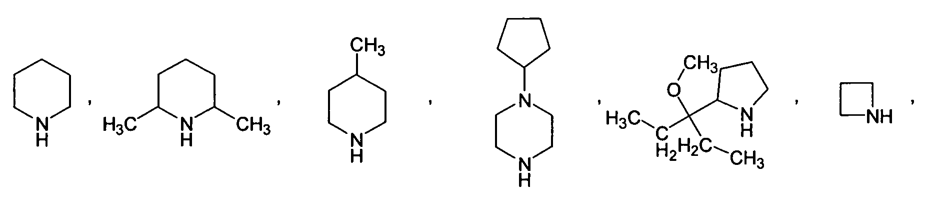 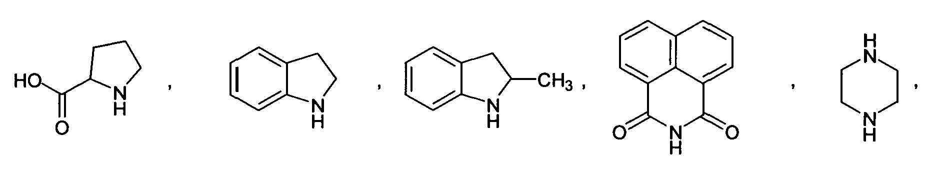 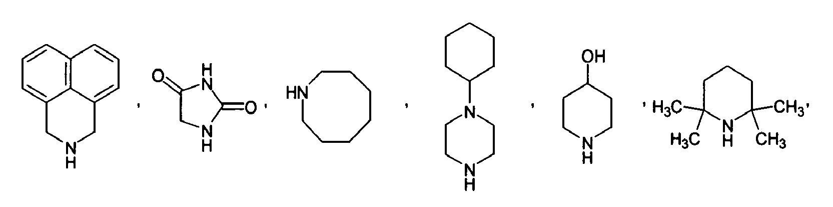 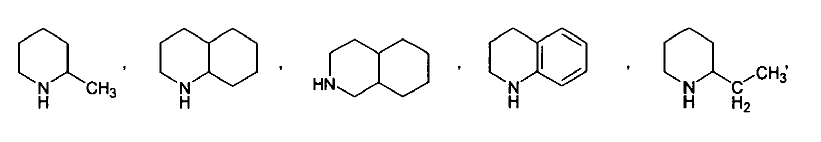 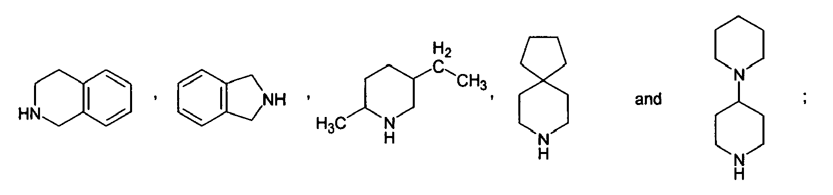 which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
more preferably R⁹ represents
a radical selected from the group consisting of phenyl, cyclohexyl, cyclopentyl, cycloheptyl, cyclooctyl or from the group of 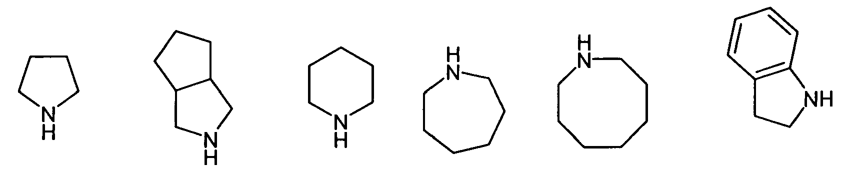 which is in each case bonded to the pyrazoline compound of general formula I at the nitrogen atom of the cyclic part of the aforementioned radicals.

In another preferred embodiment of the invention the substituted pyrazoline compounds of the invention are selected from the group consisting of:
- 5-(4-Chloro-phenyl)-1-(2,4-dichloro-phenyl)-5-methyl-4,5-dihydro-1H-pyrazole-3-carboxylic acid piperidin-1-ylamide; hydrochloride;
- 5-(4-Chloro-phenyl)-1-(2,4-dichloro-phenyl)-5-methyl-4,5-dihydro-1H-pyrazole-3-carboxylic acid azepan-1-ylamide; hydrochloride;
- 5-(4-Chloro-phenyl)-1-(2,4-dichloro-phenyl)-5-methyl-4,5-dihydro-1H-pyrazole-3-carboxylic acid cycloheptylamide;
optionally in the form of its racemate, pure stereoisomers, especially enantiomers or diastereomers or in the form of mixtures of stereoisomers, especially enantiomers or diastereomers, in any suitable ratio;
optionally in the form of a corresponding N-oxide, a corresponding salt or a corresponding solvate.

In another preferred embodiment of the invention the substituted pyrazoline compounds of the invention are compounds according to general formula V 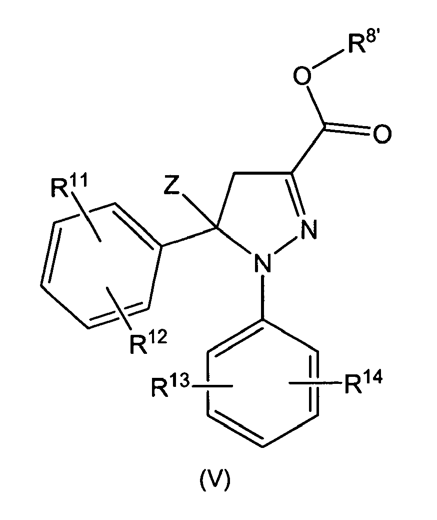 wherein
Z is C₁₋₄-Alkyl, substituted or unsubstituted, branched or linear, saturated or unsaturated;
R^{8'} represents a hydrogen atom or a branched or linear C₁₋₄-alkyl group;
R¹¹, R¹², R¹³ and R¹⁴ independently of one another represent:
   H; branched or linear C₁₋₃-alkyl or branched or linear C₁₋₃-alkoxy, phenyl, hydroxy, chloro, bromo, fluoro, iodo, SH, trifluoromethyl, CHF₂, CH₂F, OCHF_{2,} trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

As the general formula V also covers the enantiomers it may also be in the form of general formulas Va and Vb.

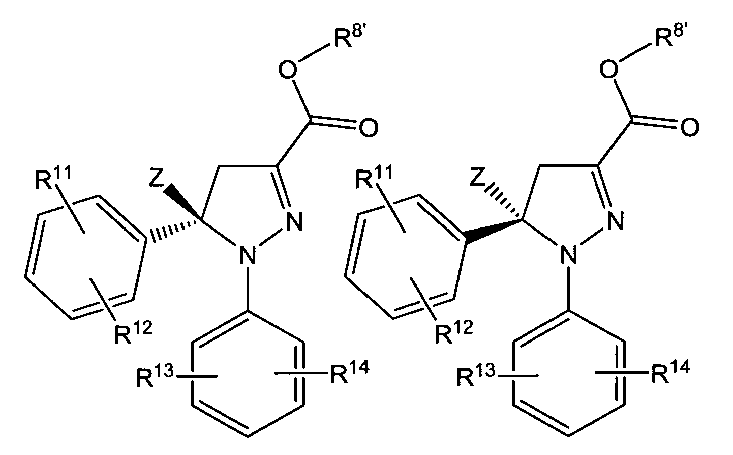

In another preferred embodiment of the invention the substituted pyrazoline compounds of the invention are compounds according to general formula V, wherein
R¹¹, R¹², R¹³ and R¹⁴ independently of one another represent H, CH₃, C₂H₅, C₃H₇, OCH₃, OC₂H₅, OH, SH, F, Cl, Br, I CF₃, CHF₂, CH₂F, OCF₃, OCHF₂; preferably R¹¹, R¹², R¹³ and R¹⁴ independently of one another represent H, OH, OCH₃, F, Cl, Br, I, CF₃, CHF₂ or OCF₃; and/or
Z is CH₃ or C₂H₅; and/or
R^{8'} represents a hydrogen atom, CH₃ or C₂H₅.

In another preferred embodiment of the invention the substituted pyrazoline compounds of the invention are compounds according to general formula VI 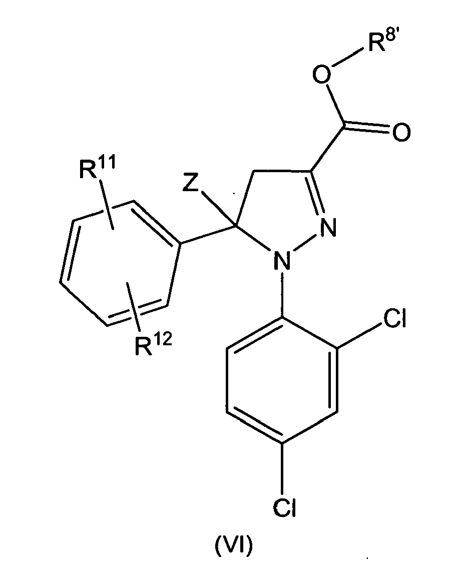 wherein
Z is C₁₋₄-Alkyl, substituted or unsubstituted, branched or linear, saturated or unsaturated;
R^{8'} represents a hydrogen atom or a branched or linear C₁₋₄-alkyl group;
R¹¹ and R¹² independently of one another represent:
   H; branched or linear C₁₋₃-alkyl or branched or linear C₁₋₃-alkoxy, phenyl, hydroxy, chloro, bromo, fluoro, iodo, SH, trifluoromethyl, CHF₂, CH₂F, OCHF₂, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

As the general formula VI also covers the enantiomers it may also be in the form of general formulas VIa and Vlb.

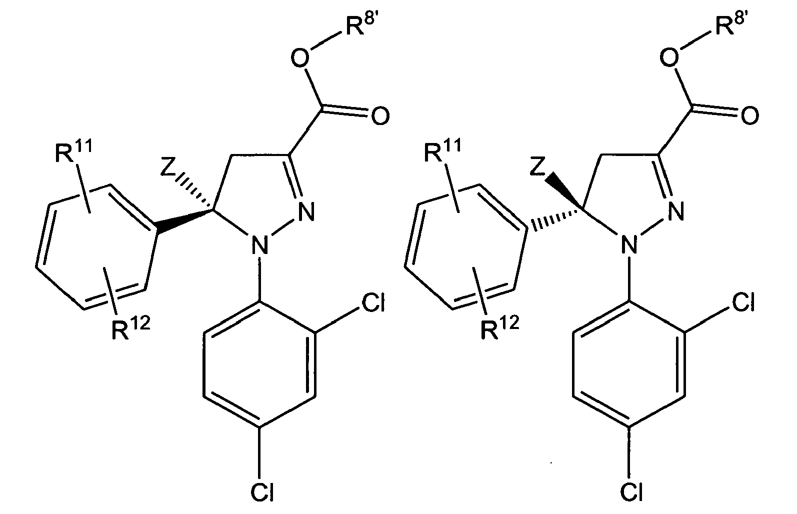

In another preferred embodiment of the invention the substituted pyrazoline compounds of the invention are compounds according to general formula VI, wherein
R¹¹ and R¹² independently of one another represent H, CH₃, C₂H₅, C₃H₇, OCH₃, OC₂H₅, OH, SH, F, CI, Br, I CF₃, CHF₂, CH₂F, OCF₃, OCHF₂; preferably represent H, OH, OCH₃, F, Cl, Br, I, CF₃, CHF₂ or OCF₃; more preferably represent Br, Cl, OH, OCH₃, or H; most preferably represent H; and/or
Z is CH₃ or C₂H₅; preferably is CH₃; and/or
R^{8'} is hydrogen, CH₃ or C₂H₅; preferably is hydrogen or C₂H₅.

In another preferred embodiment of the invention the substituted pyrazoline compounds of the invention are compounds selected from the group consisting of:
- 5-(4-Chloro-phenyl)-1-(2,4-dichloro-phenyl)-5-methyl-4,5-dihydro-1 H-pyrazole-3-carboxylic acid;
- 5-(4-Chloro-phenyl)-1-(2,4-dichloro-phenyl)-5-methyl-4,5-dihydro-1 H-pyrazole-3-carboxylic acid ethyl ester;
optionally in the form of its racemate, pure stereoisomers, especially enantiomers or diastereomers or in the form of mixtures of stereoisomers, especially enantiomers or diastereomers, in any suitable ratio; optionally in the form of a corresponding N-oxide, a corresponding salt or a corresponding solvate.

The afore mentioned reactions involving the synthesis of the 4,5-dihydro-pyrazole ring or the reaction of a compound comprising said ring are carried out under an inert atmosphere, preferably nitrogen or argon, to avoid oxidation of the ring-system.

During the processes described above the protection of sensitive groups or of reagents may be necessary and/or desirable. The introduction of conventional protective groups as well as their removal may be performed by methods well-known to those skilled in the art.

If the substituted pyrazoline compounds of general formula (I) themselves are obtained in form of a mixture of stereoisomers, particularly enantiomers or diastereomers, said mixtures may be separated by standard procedures known to those skilled in the art, e.g. chromatographic methods or fractunalized crystallization with chiral reagents. It is also possible to obtain pure stereoisomers via stereoselective synthesis.

In a further aspect the present invention also provides a process for the preparation of salts of substituted pyrazoline compounds of general formula (I) and stereoisomers thereof, wherein at least one compound of general formula (I) having at least one basic group is reacted with at least one inorganic and/or organic acid, preferably in the presence of a suitable reaction medium. Suitable reaction media include, for example, any of the ones given above. Suitable inorganic acids include hydrochloric acid, hydrobromic acid, phosphoric acid, sulfuric acid, nitric acid, suitable organic acids are e.g. citric acid, maleic acid, fumaric acid, tartaric acid, or derivatives thereof, p-toluenesulfonic acid, methanesulfonic acid or camphersulfonic acid.

In yet a further aspect the present invention also provides a process for the preparation of salts of substituted pyrazoline compounds of general formula (I) or stereoisomers thereof, wherein at least one compound of general formula (I) having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. [NHₙR₄₋ₙ]⁺, wherein n is 0, 1, 2, 3 or 4 and R represents a branched or unbranched C₁₋₄-alkyl-radical. Suitable reaction media are, for example, any of the ones given above.

Solvates, preferably hydrates, of the substituted pyrazoline compounds of general formula (I), of corresponding stereoisomers, of corresponding N-oxides or of corresponding salts thereof may also be obtained by standard procedures known to those skilled in the art.

Substituted pyrazoline compounds of general formula I, which comprise nitrogen-atom containing saturated, unsaturated or aromatic rings may also be obtained in the form of their N-oxides by methods well known to those skilled in the art.

Those skilled in the art understand that the term substituted pyrazoline compounds as used herein is to be understood as encompassing derivatives such as ethers, esters and complexes of these compounds as well. The term "derivatives" as used in this application is defined here as meaning a chemical compound having undergone a chemical derivation starting from an acting (active) compound to change (ameliorate for pharmaceutical use) any of its physico-chemical properties, especially a so-called prodrug, e.g. their esters and ethers. Examples of well known methods of producing a prodrug of a given acting compound are known to those skilled in the art and can be found e.g. in Krogsgaard-Larsen et al., Textbook of Drugdesign and Discovery, Taylor & Francis (April 2002). The respective description is hereby incorporated by reference and forms part of the disclosure.

The purification and isolation of the inventive substituted pyrazoline compounds of general formula (I), of a corresponding stereoisomer, or salt, or N-oxide, or solvate or any intermediate thereof may, if required, be carried out by conventional methods known to those skilled in the art, e.g. chromatographic methods or recrystallization.

The substituted pyrazoline compounds of general formula I given above, their stereoisomers, corresponding N-oxides, corresponding salts thereof and corresponding solvates are toxicologically acceptable and are therefore suitable as pharmaceutical active substances for the preparation of medicaments.

Thus, an other aspect of the present invention relates to a medicament comprising at least, optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof, and optionally at least one physiologically acceptable auxiliary agent.

Another aspect of the present invention is the use of at least one substituted pyrazoline compound of general formula I given above as suitable active substances, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients, for the preparation of a medicament for for the prophylaxis and/or treatment of dyslipidaemia; diabetes Type II, Metabolic Syndrome or obesity; especially dyslipidaemia.

Also particularly preferred is the use of at least one of the pyrazoline compounds as defined herein and optionally one or more pharmaceutically acceptable excipients, for the preparation of a medicament for the prophylaxis and/or treatment of dyslipidaemia.

Also particularly preferred is the use of at least one of the pyrazoline compounds as defined herein and optionally one or more pharmaceutically acceptable excipients, for the preparation of a medicament for the prophylaxis and/or treatment of metabolic syndrome, preferably also the weight independent aspects of metabolic syndrome.

Also particularly preferred is the use of at least one of the pyrazoline compounds as defined herein and optionally one or more pharmaceutically acceptable excipients, for the preparation of a medicament for the prophylaxis and/or treatment of diabetes Type II.

Also particularly preferred is the use of at least one of the pyrazoline compounds as defined herein and optionally one or more pharmaceutically acceptable excipients, for the preparation of a medicament for the prophylaxis and/or treatment of obesity.

Also particularly preferred is the use of at least one of the respective substituted pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients, for the preparation of a medicament for the prophylaxis and/or treatment of food intake disorders, preferably bulimia, anorexia, cachexia, obesity and/or type II diabetus mellitus (non-insuline dependent diabetes mellitus), more preferably obesity.

Another aspect of the present invention is a method of treating dyslipidaemia; diabetes Type II, Metabolic Syndrome or obesity; especially dyslipidaemia in a patient in need thereof with at least one substituted pyrazoline compound of general formula I given above as suitable active substances, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients.dyslipidaemia; diabetes Type II, Metabolic Syndrome or obesity; especially dyslipidaemia.

The medicament according to the present invention may be in any form suitable for the application to humans and/or animals, preferably humans including infants, children and adults and can be produced by standard procedures known to those skilled in the art. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or retarded release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments according to the present invention may also comprise an enteric coating, so that their dissolution is dependent on pH-value. Due to said coating the medicament can pass the stomach undissolved and the respective nitro-subsituted phenyl-piperazine compound is liberated in the intestinal tract. Preferably the enteric coating is soluble at a pH value of 5 to 7.5. Suitable materials and methods for the preparation are known from the

### prior art.

Typically, the medicaments according to the present invention may contain 1-60 % by weight of one or more substituted pyrazoline compounds as defined herein and 40-99 % by weight of one or more auxiliary substances (additives).

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000, even more preferably 1 to 150 milligrams of active substance to be administered during one or several intakes per day.

The present invention is illustrated below with the aid of examples. These illustrations are given solely by way of example and do not limit the general spirit of the present invention.

The present invention is illustrated below with the aid of examples. These illustrations are given solely by way of example and do not limit the general spirit of the present invention.

### Examples:

### CHEMICAL PART

The compounds were prepared following the general approach set out in Scheme 1:

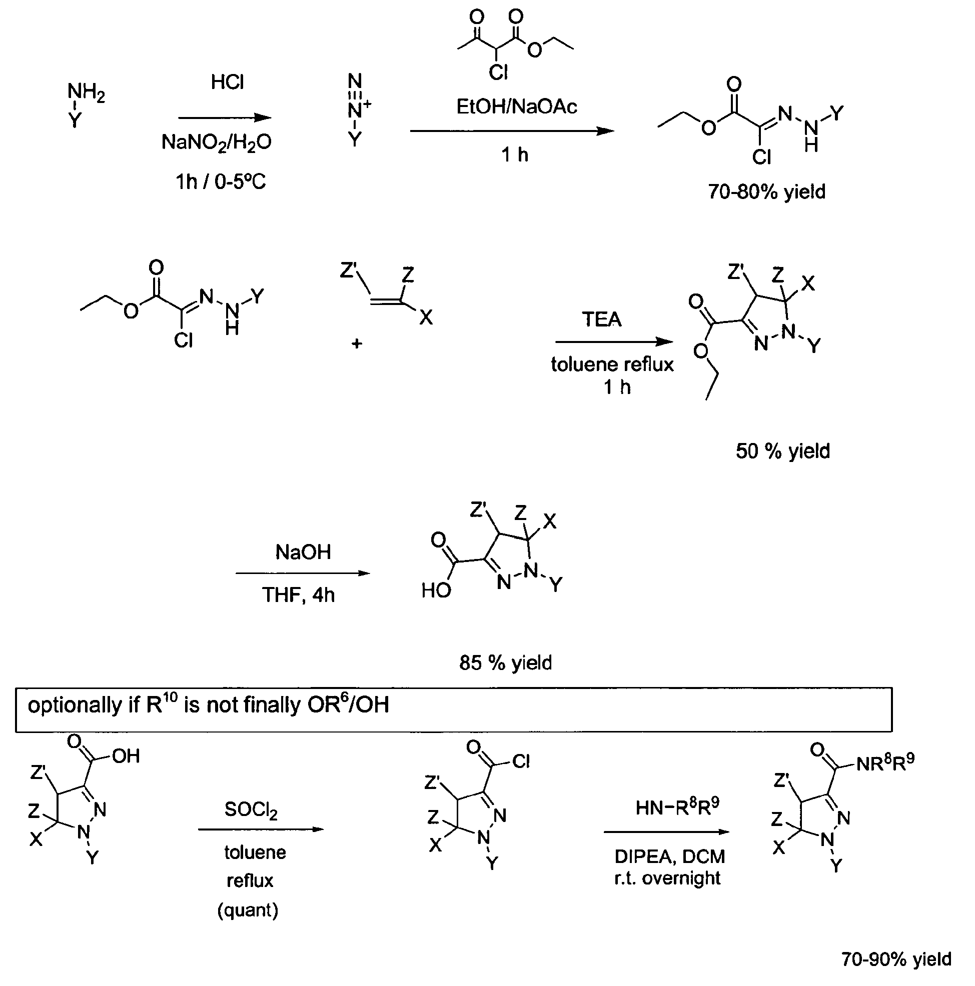

In a more specific way the chemical examples were generally prepared as follows:

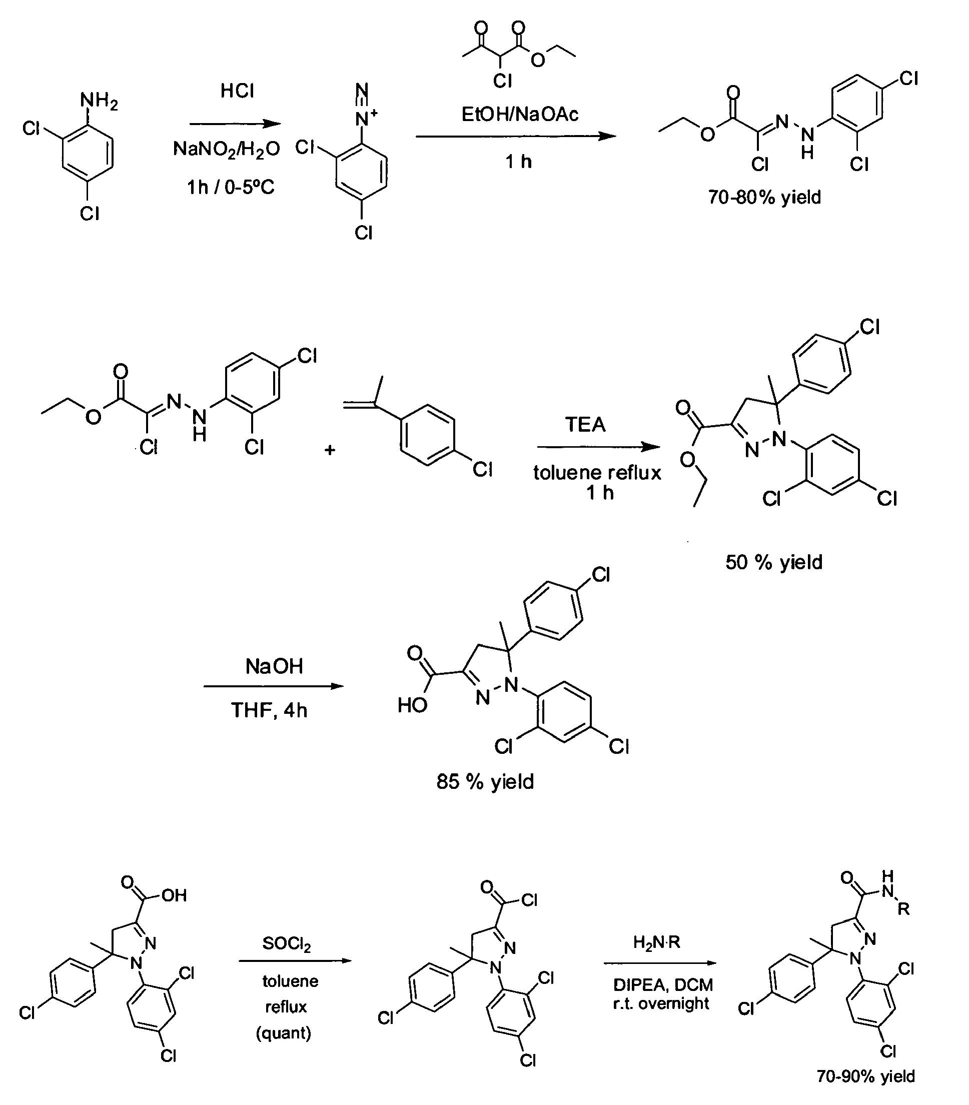

The following compounds were prepared according to the general processes described above. Those skilled in the art are familiar with the starting materials that are needed to obtain said compounds.

### Example 1: 5-(4-Chloro-phenyl)-1-(2,4-dichloro-phenyl)-5-methyl-4,5-dihydro-1H-pyrazole-3-carboxylic acid cycloheptylamide

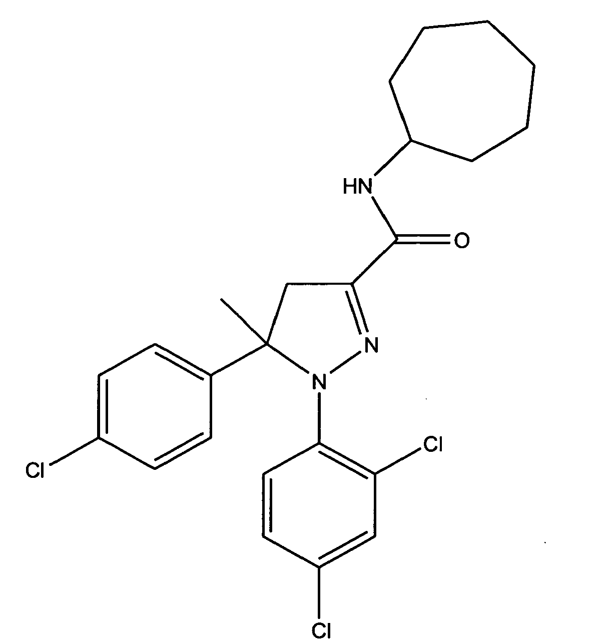

1H NMR (300 MHz, DMSO-*d*₆) δ ppm 1.34 - 1.65 (m, 12 H) 1.75 (s., 3 H) 3.30 (d, *J*=2.20 Hz, 2 H) 3.84 (m, 1 H) 6.79 (d, *J*=8.79 Hz, 1 H) 7.26 (dd, *J*=8.64, 2.34 Hz, 1 H) 7.40 (s, 4 H) 7.54 (d, *J*=2.49 Hz, 1 H) 7.87 (d, *J*=8.20 Hz, 1 H)
MS (M+H, APCI)⁺: 478

### Example 2: 5-(4-Chloro-phenyl)-1-(2,4-dichloro-phenyl)-5-methyl-4,5-dihydro-1H-pyrazole-3-carboxylic acid azepan-1-ylamide; hydrochloride

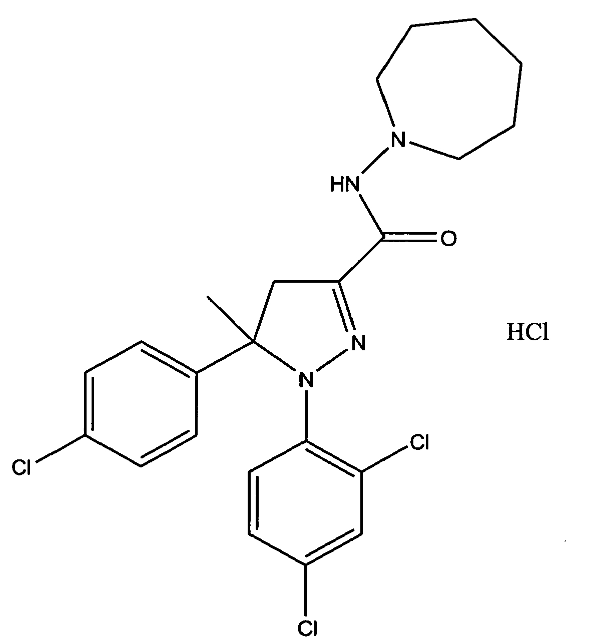

1H NMR (300 MHz, DMSO-*d*₆) δ ppm 1.59 (br. s., 8 H) 1.78 (s., 3 H) 3.38 (m, 6 H) 6.77 (d, *J*=8.79 Hz, 1 H) 7.29 (dd, *J*=8.64, 2.49 Hz, 1 H) 7.43 (s, 4 H) 7.60 (d, *J*=2.34 Hz, 1H) 11.01(br. s., 1H)
MS (M+H, APCI)⁺: 479

### Example 3: 5-(4-Chloro-phenyl)-1-(2,4-dichloro-phenyl)-5-methyl-4,5-dihydro-1H-pyrazole-3-carboxylic acid piperidin-1-ylamide; hydrochloride

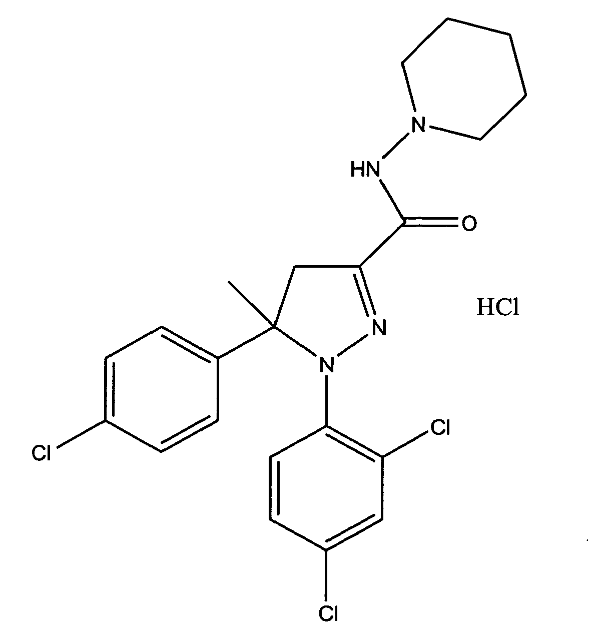

1H NMR (300 MHz, DMSO-*d*₆) δ ppm 1.42 (br. s., 2 H) 1.59 (s, 3 H) 1.73 (br. s., 4 H) 3.15 (br. s., 4 H) 3.37 (s, 2 H) 6.78 (d, *J*=8.64 Hz, 1 H) 7.29 (dd, *J*=8.64, 2.34 Hz, 1 H) 7.43 (s, 4 H) 7.59 (d, *J*=2.49 Hz, 1 H) 10.58 (br. s., 1 H)
MS (M+H, APCI)⁺: 465

### Example 4: 5-(4-Chloro-phenyl)-1-(2,4-dichloro-phenyl)-5-methyl-4,5-dihydro-1H-pyrazole-3-carboxylic acid

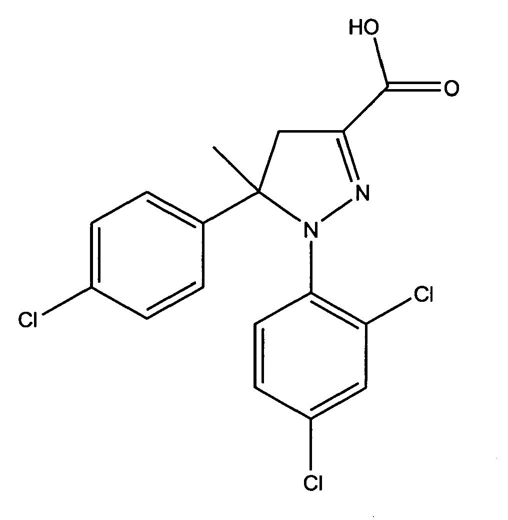

1H NMR (300 MHz, CHLOROFORM-*d*) δ ppm 1.69 (s, 3 H) 3.41 (d, *J*=3.81 Hz, 2 H) 6.46 (d, *J*=8.64 Hz, 1 H) 6.97 (dd, *J*=8.64, 2.34 Hz, 1 H) 7.30 - 7.42 (m, 2 H) 7.35 (d, *J*=4.98 Hz, 3 H)
MS (M+H, APCI)⁺: 383

### Example 5: 5-(4-Chloro-phenyl)-1-(2,4-dichloro-phenyl)-5-methyl-4,5-dihydro-1H-pyrazole-3-carboxylic acid ethyl ester

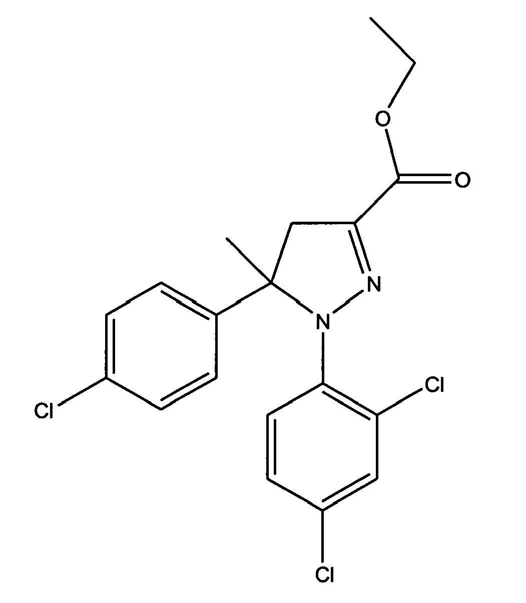

1H NMR (300 MHz, CHLOROFORM-*d*) δ ppm 1.37 (t, *J*=7.03 Hz, 3 H) 1.66 (s, 3 H) 3.39 (d, *J*=2.78 Hz, 2 H) 4.35 (q, *J*=7.03 Hz, 2 H) 6.49 (d, *J=8.64* Hz, 1 H) 6.95 (dd, *J=8.64,* 2.34 Hz, 1 H) 7.30 - 7.39 (m, 5 H)
MS (M+H, APCI)⁺: 411

### Pharmacological Data:

### Pharmacological Methods

### I. In-vitro determination of inhibition of ACAT (Acyl CoA-Cholesterol Acyltransferase)

The in-vitro determination of the inhibition of the enzyme ACAT by the compounds of the invention is carried out in principle as described in the publication of Largis EE, Wang CH, DeVries VG and Schaffer SA (1989), "A Novel inhibitor of ACAT-catalyzed cholesterol esterification and cholesterol absorption", J.Lipid Res. 30, 681-689, (1989), whereby hepatic microsomes are used. The radio-substrate used is [¹⁴C]-Palmitoyl CoA. The respective parts of the description are hereby incorporated by reference and forms part of the present disclosure.

Briefly hepatic microsomes were prepared from the Wistar rats. Enzyme inhibition experiments were performed in presence of 18 µM [¹⁴C]-Palmitoyl CoA as substrate. The vehicle used was 1 % DMSO. Compounds to be tested were present at various concentrations and the incubation buffer used was 0.2 M Phosphate buffer, pH 7.4 at 25°C. After a 15 minutes preincubation period at 37°C an incubation period of 10 minutes at 37°C followed. Quantification of [¹⁴C]Cholesterol ester was done by column chromatography. The reference compound used was Lovastatin, which has a reported IC₅₀ of 29 µM.

### Results:

The inhibition of the enzyme ACAT by the inventive substituted pyrazoline compounds was determined as described above. Some of the inhibition data and IC₅₀-values obtained are given in the table below:

| **Example** | **ACAT Inhibition (%) at various concentration of example** | | | | | | **ACAT IC50 [µM}** |
|---|---|---|---|---|---|---|---|
| | **10⁻⁴ M** | **3x10⁻⁵ M** | **10⁻⁵ M** | **3x10⁻⁶ M** | **10⁻⁵ M** | **3x10⁻⁶ M** | |
| **2** | 98 | 94 | 75 | 37 | - | - | 4.39 |
| **3** | 98 | 85 | 62 | 34 | - | - | 5.96 |
| **Lovastatin** | | | | | | | 12.5 |

## Claims

1. Substituted pyrazoline compound of general formula I, 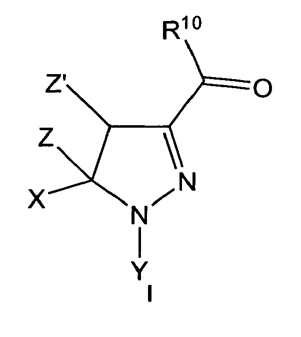 wherein
Z is C₁₋₄-Alkyl, substituted or unsubstituted, branched or linear, saturated or unsaturated;
Z' is selected from hydrogen; C₁₋₄-Alkyl, substituted or unsubstituted, branched or linear, saturated or unsaturated;
X and Y independently represent an optionally at least monsubstituted mono- or polycyclic ring-system;
R¹⁰ represents OR^{8'} or NR⁸R⁹, with
R^{8'} representing a hydrogen atom or a branched or linear C₁₋₄-alkyl group, or
either
R⁸ representing a hydrogen atom or a branched or linear C₁₋₃-alkyl group, while R⁹ is representing an optionally at least monsubstituted mono- or polycyclic ring-system;
or
R⁸ and R⁹ together with the connecting Nitrogen atom are representing an optionally at least monsubstituted heterocyclyl radical;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

2. Substituted pyrazoline compound according to claim 1 according to general formula I, wherein
Z is C₁₋₄-Alkyl, substituted or unsubstituted, branched or linear, saturated or unsaturated;
Z' is selected from hydrogen; C₁₋₄-Alkyl, substituted or unsubstituted, branched or linear, saturated or unsaturated;
X and Y independently represent an aryl radical, cycloalkyl radical, or heterocyclyl radical, which groups are unsubstituted or may be substituted with 1, 2 or 3 substituents W, which can be the same or different, selected from the group
branched or linear C₁₋₃-alkyl or branched or linear C₁₋₃-alkoxy, phenyl, hydroxy, chloro, bromo, fluoro, iodo, SH, trifluoromethyl, CHF₂, CH₂F, OCHF₂, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
R¹⁰ represents OR^{8'} or NR⁸R⁹, with
R^{8'} representing a hydrogen atom or a branched or linear C₁₋₄-alkyl group, or
either
R⁸ representing a hydrogen atom or a branched or linear C₁₋₃-alkyl group, while R⁹ is representing an aryl radical, cycloalkyl radical, or heterocyclyl radical, which groups are unsubstituted or may be substituted with; R⁵, R⁶ and R⁷, which can be the same or different,
with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂;
or
R⁸ and R⁹ together with the connecting Nitrogen atom are representing an optionally at least monsubstituted heterocyclyl radical; which group is unsubstituted or may be substituted with R⁵, R⁶ and R⁷, which can be the same or different,
with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

3. Substituted pyrazoline compound according to any of claims 1 or 2 according to general formula I, wherein
Z is C₁₋₄-Alkyl, substituted or unsubstituted, branched or linear, saturated or unsaturated;
Z' is selected from hydrogen; C₁₋₄-Alkyl, substituted or unsubstituted, branched or linear, saturated or unsaturated;
X and Y independently represent an phenyl, thienyl, naphtyl or pyridyl, which groups are unsubstituted or may be substituted with 1, 2 or 3 substituents W, which can be the same or different, selected from the group
branched or linear C₁₋₃-alkyl or branched or linear C₁₋₃-alkoxy, phenyl, hydroxy, chloro, bromo, fluoro, iodo, SH, trifluoromethyl, CHF₂, CH₂F, OCHF₂, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
R¹⁰ represents OR 8' or NR⁸R⁹, with
R^{8'} representing a hydrogen atom or a branched or linear C₁₋₄-alkyl group, or
either
R⁸ representing a hydrogen atom or a branched or linear C₁₋₃-alkyl group, while R⁹ is representing an aryl radical, cycloalkyl radical, or heterocyclyl radical, which groups are unsubstituted or may be substituted with; R⁵, R⁶ and R⁷, which can be the same or different,
with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂;
or
R⁸ and R⁹ together with the connecting Nitrogen atom are representing an optionally at least monsubstituted heterocyclyl radical; which group is unsubstituted or may be substituted with R⁵, R⁶ and R⁷, which can be the same or different,
with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

4. Substituted pyrazoline compound according to any of claims 1 to 3, wherein
Z is CH₃ or C₂H₅; and/or
Z' is hydrogen; and/or
X and Y independently represent phenyl or thienyl; preferably Y representing phenyl, while X represents phenyl or thienyl; more preferably X and Y representing phenyl; and/or
R¹⁰ represents OR^{8'} with
R^{8'} representing a hydrogen atom or a branched or linear C₁₋₄-alkyl group, preferably hydrogen, CH₃ or C₂H₅;
or
R¹⁰ represents NR⁸R⁹, with
R⁸ representing a hydrogen atom or a branched or linear C₁₋₃-alkyl group, preferably hydrogen; and
R⁹ is representing an aryl radical, cycloalkyl radical, or heterocyclyl radical;
or
R⁸ and R⁹ together with the connecting Nitrogen atom are representing an optionally at least monsubstituted heterocyclyl radical.

5. Substituted pyrazoline compound according to any of claims 1 to 4 **characterized in that** the compound is a compound of general formula II 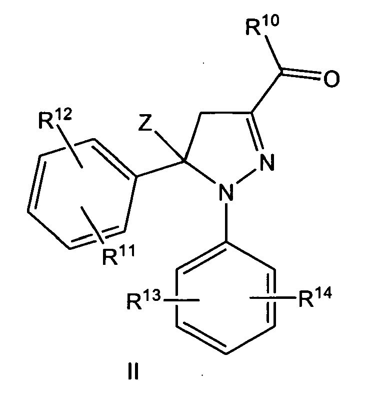 wherein
Z is C₁₋₄-Alkyl, substituted or unsubstituted, branched or linear, saturated or unsaturated;
R¹⁰ represents OR^{8'} or NR⁸R⁹, with
R^{8'} representing a hydrogen atom or a branched or linear C₁₋₄-alkyl group, or
either
R⁸ representing a hydrogen atom or a branched or linear C₁₋₃-alkyl group, while R⁹ is representing an aryl radical, cycloalkyl radical, or heterocyclyl radical, which groups are unsubstituted or may be substituted with; R⁵, R⁶ and R⁷, which can be the same or different,
with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂;
or
R⁸ and R⁹ together with the connecting Nitrogen atom are representing an optionally at least monsubstituted heterocyclyl radical; which group is unsubstituted or may be substituted with R⁵, R⁶ and R⁷, which can be the same or different,
with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂;
R¹¹, R¹², R¹³ and R¹⁴ independently of one another represent:
H; branched or linear C₁₋₃-alkyl or branched or linear C₁₋₃-alkoxy, phenyl, hydroxy, chloro, bromo, fluoro, iodo, SH, trifluoromethyl, CHF₂, CH₂F, OCHF₂, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

6. Substituted pyrazoline compound according to claim 5, wherein
R¹¹, R¹², R¹³ and R¹⁴ independently of one another represent H, CH₃, C₂H₅, C₃H₇, OCH₃, OC₂H₅, OH, SH, F, Cl, Br, I CF₃, CHF₂, CH₂F, OCF₃, OCHF₂; preferably R¹¹, R¹², R¹³ and R¹⁴ independently of one another represent H, OH, OCH₃, F, Cl, Br, I, CF₃, CHF₂ or OCF₃; and/or
Z is CH₃ or C₂H₅; and/or
R¹⁰ represents OR^{8'} with
R^{8'} representing a hydrogen atom or a branched or linear C₁₋₄-alkyl group, preferably hydrogen, CH₃ or C₂H₅;
or
R¹⁰ represents NR⁸R⁹, with
R⁸ representing a hydrogen atom or a branched or linear C₁₋₃-alkyl group, preferably hydrogen; and
R⁹ is representing an aryl radical, cycloalkyl radical, or heterocyclyl radical;
or
R⁸ and R⁹ together with the connecting Nitrogen atom are representing an optionally at least monsubstituted heterocyclyl radical.

7. Substituted pyrazoline compound according to any of claims 1 to 6, **characterized in that** the compound is a compound of general formula III 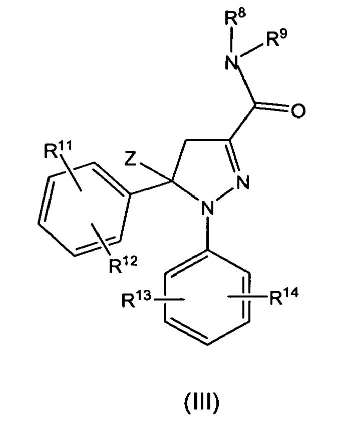 wherein
Z is C₁₋₄-Alkyl, substituted or unsubstituted, branched or linear, saturated or unsaturated;
R⁸ represents a hydrogen atom or a branched or linear C₁₋₃-alkyl group, while R⁹ is representing an aryl radical, cycloalkyl radical, or heterocyclyl radical, which groups are unsubstituted or may be substituted with; R⁵, R⁶ and R⁷, which can be the same or different,
with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂;
or
R⁸ and R⁹ together with the connecting Nitrogen atom are representing an optionally at least monsubstituted heterocyclyl radical; which group is unsubstituted or may be substituted with R⁵, R⁶ and R⁷, which can be the same or different,
with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂;
R¹¹, R¹², R¹³ and R¹⁴ independently of one another represent:
H; branched or linear C₁₋₃-alkyl or branched or linear C₁₋₃-alkoxy, phenyl, hydroxy, chloro, bromo, fluoro, iodo, SH, trifluoromethyl, CHF₂, CH₂F, OCHF₂, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

8. Substituted pyrazoline compound according to claim 7, wherein
R¹¹, R¹², R¹³ and R¹⁴ independently of one another represent H, CH₃, C₂H₅, C₃H₇, OCH₃, OC₂H₅, OH, SH, F, Cl, Br, I CF₃, CHF₂, CH₂F, OCF₃, OCHF₂; preferably R¹¹, R¹², R¹³ and R¹⁴ independently of one another represent H, OH, OCH₃, F, Cl, Br, I, CF₃, CHF₂ or OCF₃; and/or
Z is CH₃ or C₂H₅; and/or
R⁸ represents a hydrogen atom;
R⁹ represents an aryl radical, cycloalkyl radical, or heterocyclyl radical, which groups are unsubstituted or may be substituted with; R⁵, R⁶ and R⁷, which can be the same or different,
with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂.

9. Substituted pyrazoline compound according to any of claims 1 to 8 **characterized in that** the compound is a compound of general formula IV 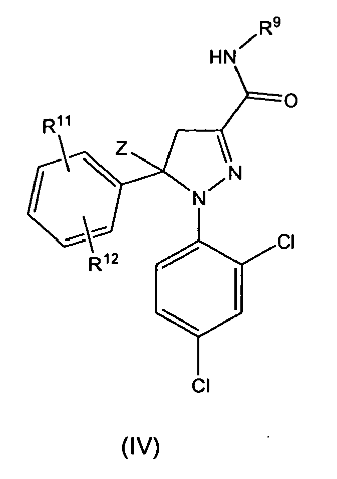 wherein
Z is C₁₋₄-Alkyl, substituted or unsubstituted, branched or linear, saturated or unsaturated;
R⁹ represents an aryl radical, cycloalkyl radical, or heterocyclyl radical, which groups are unsubstituted or may be substituted with; R⁵, R⁶ and R⁷, which can be the same or different,
with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂;
R¹¹ and R¹² independently of one another represent:
H; branched or linear C₁₋₃-alkyl or branched or linear C₁₋₃-alkoxy, phenyl, hydroxy, chloro, bromo, fluoro, iodo, SH, trifluoromethyl, CHF₂, CH₂F, OCHF_{2,} trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

10. Substituted pyrazoline compound according to claim 9, wherein
R¹¹ and R¹² independently of one another represent H, CH₃, C₂H₅, C₃H₇, OCH₃, OC₂H₅, OH, SH, F, Cl, Br, I CF₃, CHF₂, CH₂F, OCF₃, OCHF₂; preferably R¹¹, R¹², R¹³ and R¹⁴ independently of one another represent H, OH, OCH₃, F, Cl, Br, I, CF₃, CHF₂ or OCF₃; and/or
Z is CH₃ or C₂H₅; and/or
R⁹ represents an aryl radical, cycloalkyl radical, or heterocyclyl radical, which groups are unsubstituted or may be substituted with; R⁵, R⁶ and R⁷, which can be the same or different,
with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂.

11. Substituted pyrazoline compound according to claim 9, wherein
R¹¹ and R¹² independently of one another represent H, CH₃, C₂H₅, C₃H₇, OCH₃, OC₂H₅, OH, SH, F, Cl, Br, I CF₃, CHF₂, CH₂F, OCF₃, OCHF₂; preferably represent H, OH, OCH₃, F, Cl, Br, I, CF₃, CHF₂ or OCF₃; more preferably represent Br, Cl, OH, OCH₃, or H; most preferably represent H; and/or
Z is CH₃ or C₂H₅; preferably is CH₃; and/or
R⁹ represents an aryl radical, cycloalkyl radical, or heterocyclyl radical, which groups are unsubstituted or may be substituted with; R⁵, R⁶ and R⁷, which can be the same or different,
with R⁵, R⁶ and R⁷ being independently from one another selected from H, F, Cl, Br, I, OH, SH, C₁₋₄alkyl, C₁₋₄alkoxy, CF₃, CHF₂, CH₂F, OCF₃, a keto-group, NO₂ or NH₂;
preferably represents an unsubstituted aryl radical, cycloalkyl radical, or heterocyclyl radical.

12. Substituted pyrazoline compound according to claim 9, wherein
R¹¹ and R¹² independently of one another represent H, CH₃, C₂H₅, C₃H₇, OCH₃, OC₂H₅, OH, SH, F, Cl, Br, I CF₃, CHF₂, CH₂F, OCF₃, OCHF₂; preferably represent H, OH, OCH₃, F, Cl, Br, I, CF₃, CHF₂ or OCF₃; more preferably represent Br, Cl, OH, OCH₃, or H; most preferably represent H; and
Z is CH₃ or C₂H₅; preferably is CH₃; and
R⁹ represents
a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl and bicyclo[2.2.1]heptyl, which may be bonded via a -(CH₂)-, -(CH₂)-(CH₂)-, -(CH₂)-(CH₂)-(CH₂)- or -CH=CH-group and/or may optionally be substituted with 1, 2, 3, 4 or 5 substituent(s) independently selected from the group consisting of - OH, methyl, ethyl, n-propyl, isopropyl, n-butyl, isobutyl, 2-butyl, tert-butyl, n-pentyl, 2-pentyl and n-hexyl;
a radical selected from the group consisting of phenyl, naphthyl, pyridinyl, furyl (furanyl), thienyl (thiophenyl) and triazolyl, which may be bonded via a -(CH₂)-, -(CH₂)-(CH₂)-, -(CH₂)-(CH₂)-(CH₂)- or -CH=CH-group and/or may optionally be substituted with 1, 2, 3, 4 or 5 substituent(s) independently selected from the group consisting of -CF₃, methyl, ethyl, n-propyl, isopropyl, n-butyl, isobutyl, 2-butyl, tert-butyl, n-pentyl, 2-pentyl, n-hexyl, F, Cl and Br;
or a radical selected from the group consisting of 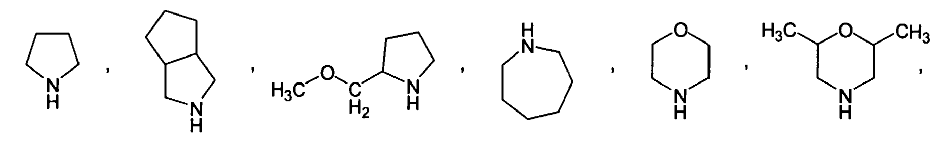 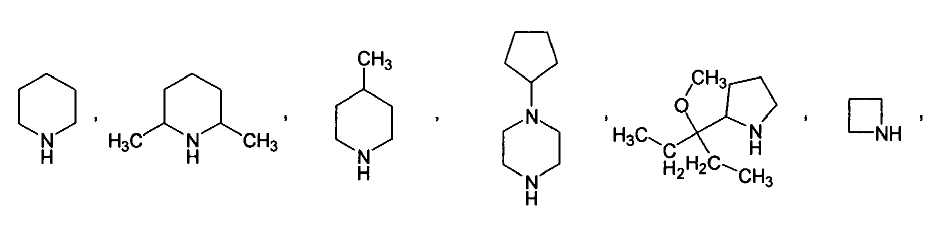 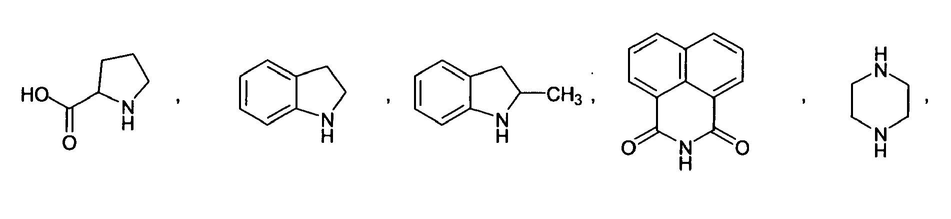 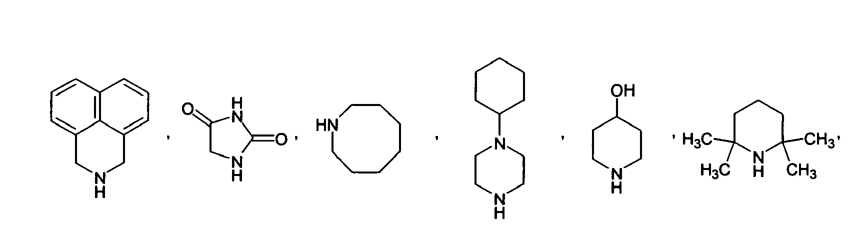 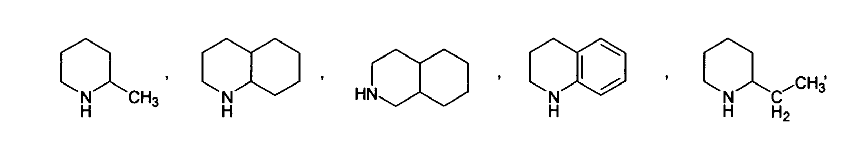 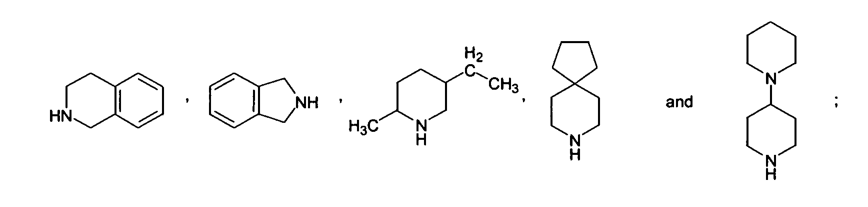 which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
preferably R⁹ represents a radical selected from the group consisting of phenyl, cyclohexyl, cyclopentyl, cycloheptyl, cyclooctyl or of the group consisting of 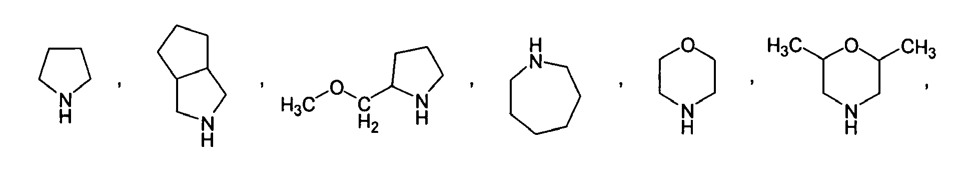 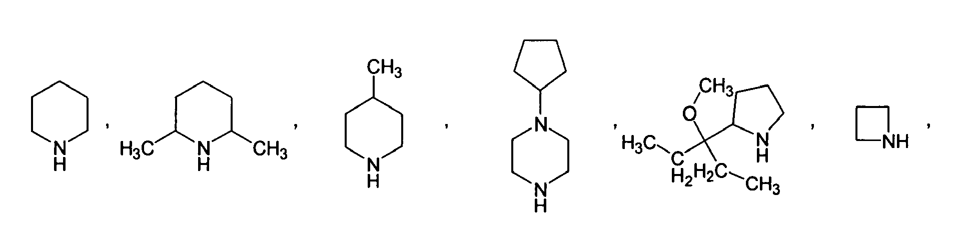 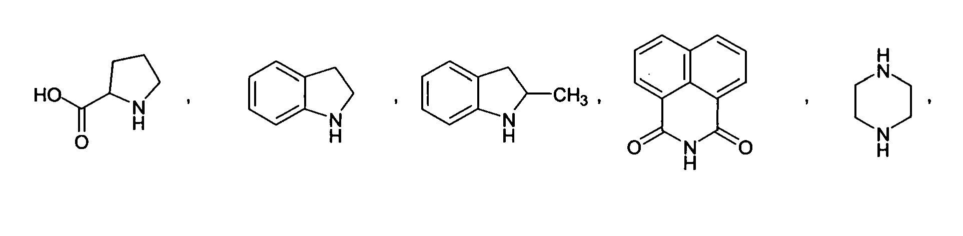 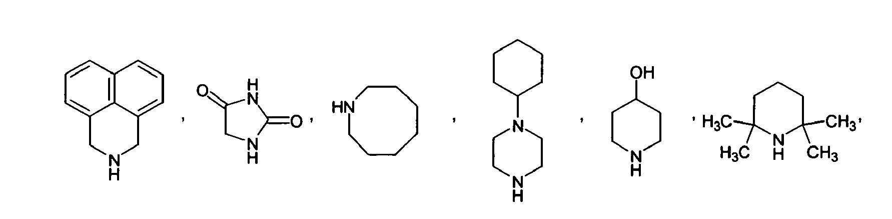 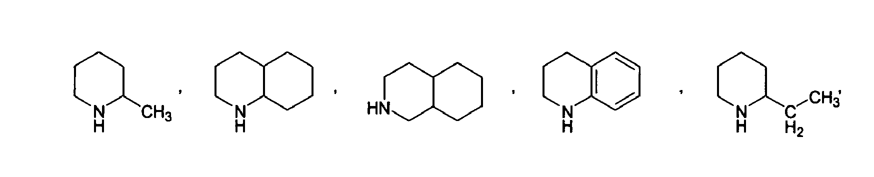 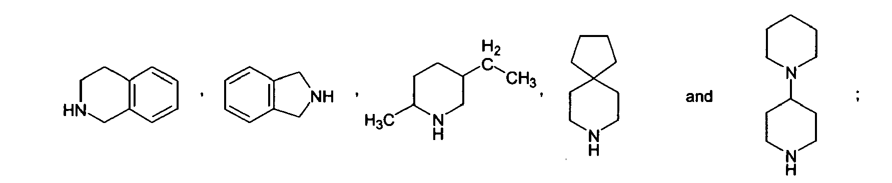 which is in each case bonded to the pyrazoline compound of general formula I at the nitrogen atom of the cyclic part of the aforementioned radicals;
more preferably R⁹ represents
a radical selected from the group consisting of phenyl, cyclohexyl, cyclopentyl, cycloheptyl, cyclooctyl or from the group of 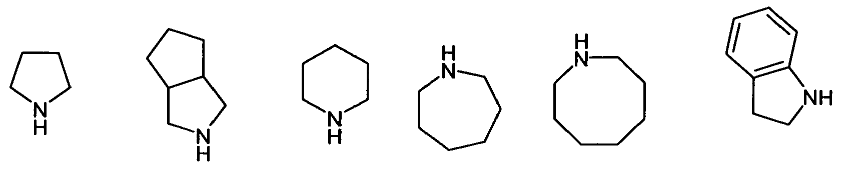 which is in each case bonded to the pyrazoline compound of general formula I at the nitrogen atom of the cyclic part of the aforementioned radicals.

13. Substituted pyrazoline compound according to any of claims 1 to 12, wherein
R⁹ represents
a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl and bicyclo[2.2.1]heptyl, which may be bonded via a -(CH₂)-, -(CH₂)-(CH₂)-, -(CH₂)-(CH₂)-(CH₂)- or -CH=CH-group and/or may optionally be substituted with 1, 2, 3, 4 or 5 substituent(s) independently selected from the group consisting of - OH, methyl, ethyl, n-propyl, isopropyl, n-butyl, isobutyl, 2-butyl, tert-butyl, n-pentyl, 2-pentyl and n-hexyl;
a radical selected from the group consisting of phenyl, naphthyl, pyridinyl, furyl (furanyl), thienyl (thiophenyl) and triazolyl, which may be bonded via a -(CH₂)-, -(CH₂)-(CH₂)-, -(CH₂)-(CH₂)-(CH₂)- or -CH=CH-group and/or may optionally be substituted with 1, 2, 3, 4 or 5 substituent(s) independently selected from the group consisting of -CF₃, methyl, ethyl, n-propyl, isopropyl, n-butyl, isobutyl, 2-butyl, tert-butyl, n-pentyl, 2-pentyl, n-hexyl, F, Cl and Br;
or a radical selected from the group consisting of 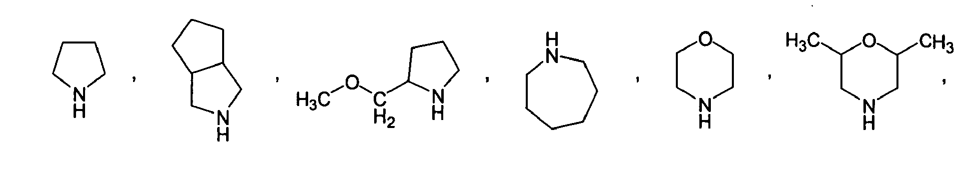 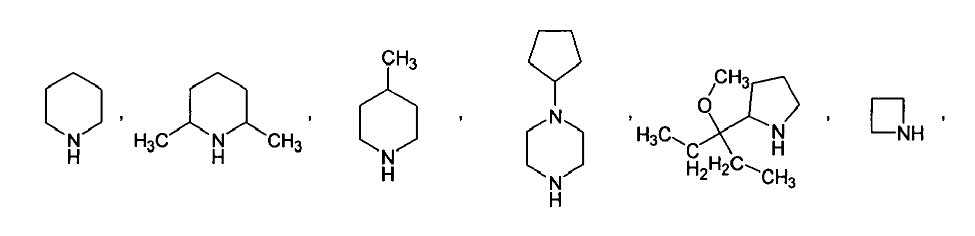 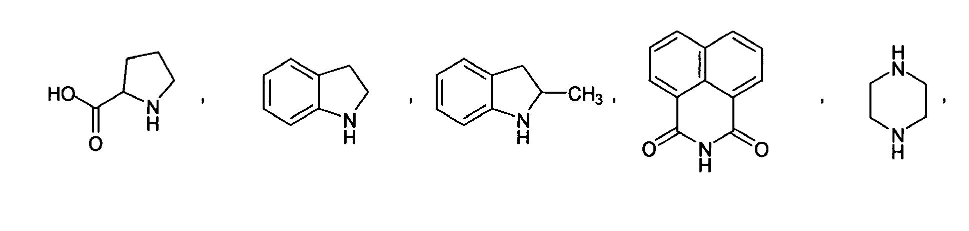 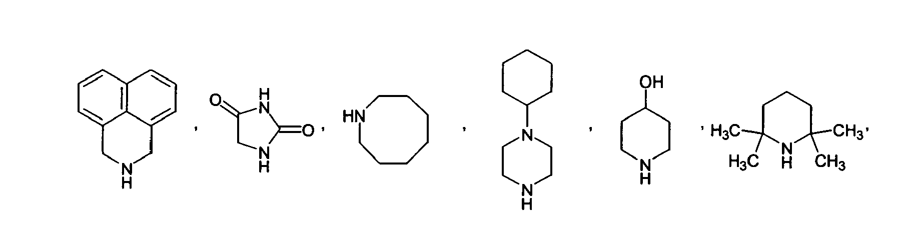 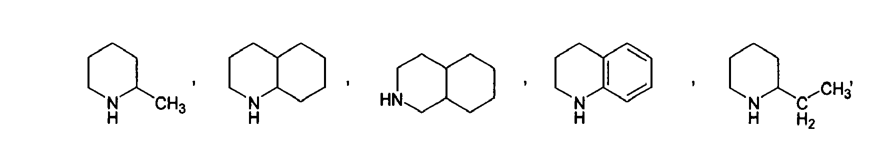 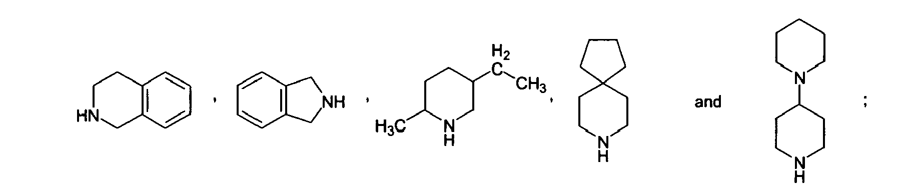 which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
more preferably R⁹ represents
a radical selected from the group consisting of phenyl, cyclohexyl, cyclopentyl, cycloheptyl, cyclooctyl or from the group of 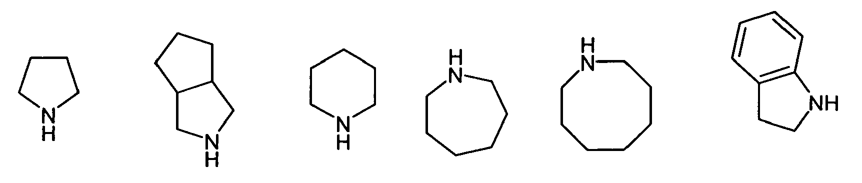
which is in each case bonded to the pyrazoline compound of general formula I at the nitrogen atom of the cyclic part of the aforementioned radicals.

14. Compound according to any of claims 1 to 13 selected from the group consisting of:
• 5-(4-Chloro-phenyl)-1-(2,4-dichloro-phenyl)-5-methyl-4,5-dihydro-1H-pyrazole-3-carboxylic acid piperidin-1-ylamide; hydrochloride;
• 5-(4-Chloro-phenyl)-1-(2,4-dichloro-phenyl)-5-methyl-4,5-dihydro-1H-pyrazole-3-carboxylic acid azepan-1-ylamide; hydrochloride;
• 5-(4-Chloro-phenyl)-1-(2,4-dichloro-phenyl)-5-methyl-4,5-dihydro-1H-pyrazole-3-carboxylic acid cycloheptylamide;
optionally in the form of its racemate, pure stereoisomers, especially enantiomers or diastereomers or in the form of mixtures of stereoisomers, especially enantiomers or diastereomers, in any suitable ratio;
optionally in the form of a corresponding N-oxide, a corresponding salt or a corresponding solvate.

15. Substituted pyrazoline compound according to any of claims 1 to 6 **characterized in that** the compound is a compound of general formula V 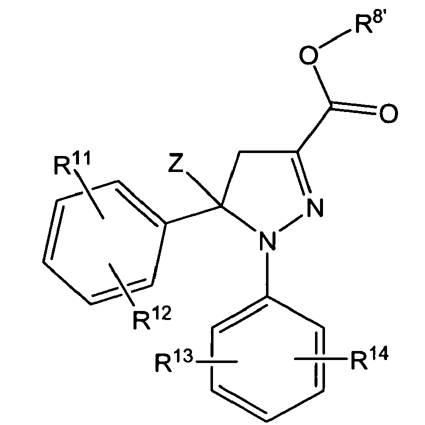 wherein
Z is C₁₋₄-Alkyl, substituted or unsubstituted, branched or linear, saturated or unsaturated;
R^{8'} represents a hydrogen atom or a branched or linear C₁₋₄-alkyl group;
R¹¹, R¹², R¹³ and R¹⁴ independently of one another represent:
H; branched or linear C₁₋₃-alkyl or branched or linear C₁₋₃-alkoxy, phenyl, hydroxy, chloro, bromo, fluoro, iodo, SH, trifluoromethyl, CHF₂, CH₂F, OCHF₂, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

16. Substituted pyrazoline compound according to claim 15, wherein
R¹¹, R¹², R¹³ and R¹⁴ independently of one another represent H, CH₃, C₂H₅, C₃H₇, OCH₃, OC₂H₅, OH, SH, F, Cl, Br, I CF₃, CHF₂, CH₂F, OCF₃, OCHF₂; preferably R¹¹, R¹², R¹³ and R¹⁴ independently of one another represent H, OH, OCH₃, F, Cl, Br, I, CF₃, CHF₂ or OCF₃; and/or
Z is CH₃ or C₂H₅; and/or
R^{8'} represents a hydrogen atom, CH₃ or C₂H₅.

17. Substituted pyrazoline compound according to claim 15, **characterized in that** the compound is a compound of general formula VI 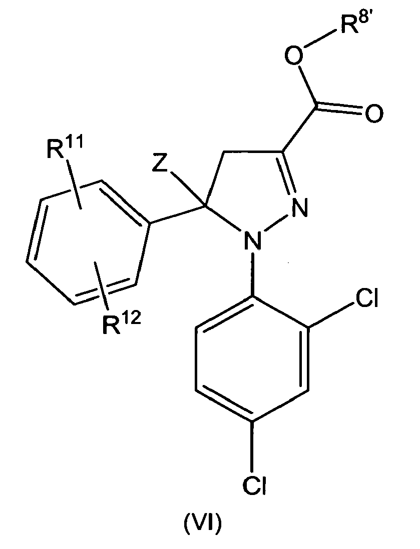 wherein
Z is C₁₋₄-Alkyl, substituted or unsubstituted, branched or linear, saturated or unsaturated;
R^{8'} represents a hydrogen atom or a branched or linear C₁₋₄-alkyl group;
R¹¹ and R¹² independently of one another represent:
H; branched or linear C₁₋₃-alkyl or branched or linear C₁₋₃-alkoxy, phenyl, hydroxy, chloro, bromo, fluoro, iodo, SH, trifluoromethyl, CHF₂, CH₂F, OCHF₂, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

18. Substituted pyrazoline compound according to claim 17, wherein
R¹¹ and R¹² independently of one another represent H, CH₃, C₂H₅, C₃H₇, OCH₃, OC₂H₅, OH, SH, F, Cl, Br, I CF₃, CHF₂, CH₂F, OCF₃, OCHF₂; preferably represent H, OH, OCH₃, F, Cl, Br, I, CF₃, CHF₂ or OCF₃; more preferably represent Br, Cl, OH, OCH₃, or H; most preferably represent H; and/or
Z is CH₃ or C₂H₅; preferably is CH₃; and/or
R^{8'} is hydrogen, CH₃ or C₂H₅; preferably is hydrogen or C₂H₅.

19. Compound according to any of claims 1 to 6 and 15 to 18 selected from the group consisting of:
• 5-(4-Chloro-phenyl)-1-(2,4-dichloro-phenyl)-5-methyl-4,5-dihydro-1H-pyrazole-3-carboxylic acid;
• 5-(4-Chloro-phenyl)-1-(2,4-dichloro-phenyl)-5-methyl-4,5-dihydro-1H-pyrazole-3-carboxylic acid ethyl ester;
optionally in the form of its racemate, pure stereoisomers, especially enantiomers or diastereomers or in the form of mixtures of stereoisomers, especially enantiomers or diastereomers, in any suitable ratio;
optionally in the form of a corresponding N-oxide, a corresponding salt or a corresponding solvate.

20. Medicament comprising at least one substituted pyrazoline compound of general formula I according to claims 1 to 19, and optionally one or more pharmaceutically acceptable excipients.

21. Use of at least one substituted pyrazoline compound according to one or more of claims 1 to 19 and optionally one or more pharmaceutically acceptable excipients, for the preparation of a medicament for the prophylaxis and/or treatment of dyslipidaemia; diabetes Type II, Metabolic Syndrome or obesity; especially dyslipidaemia.
